# EUROPEAN PATENT APPLICATION

(11) **EP 4 269 432 A1**
(43) Date of publication of application: **01.11.2023**
(21) Application number: 22305618.5
(22) Date of filing: 26.04.2022
(51) Int. Cl.: C07K 16/00, C12N 15/79

(54) **PRODUCTION OF THERAPEUTIC ANTIBODIES BY THE MICROALGAE PHAEODACTYLUM TRICORNUTUM**

(71) Applicant: Universite de Rouen Normandie, 76130 Mont-Saint-Aignan (FR)
(72) Inventor: TOUSTOU, Charlotte, 76130 MONT SAINT AIGNAN (FR); MEKHALFI, Malika, 45000 ORLEANS (FR); KIEFER-MEYER, Marie-Christine, 76130 MONT SAINT AIGNAN (FR); BARDOR, Muriel, 76230 ISNEAUVILLE (FR)
(74) Representative: Regimbeau

(57) **Abstract**

Monoclonal antibodies represent the most rapidly growing category of the recombinant therapeutic protein pipeline, with more than 85% of the therapeutic indications. The present invention proposes novel vectors that have been optimized so as to produce high levels of monoclonal antibodies when transfected in the microalgae *Phaeodactylum tricornutum,* as well as optimized culture conditions of said microalgae cells. Altogether, the present invention provides new systems for producing high amounts of monoclonal antibodies, functional fragments or derivatives thereof, in microalgae.

## Description

Monoclonal antibodies (mAbs) represent the most rapidly growing category of the recombinant therapeutic protein pipeline, with more than 85% of the therapeutic indications. In 2018, the overall sales of mAbs represented above US$115 billion and is expected to increase up to US$300 billion towards 2025. As of December 2019, 79 mAbs were granted approval by the US FDA and several hundred are still in developments or clinical trials worldwide.

mAbs are glycosylated and heterotetrameric proteins folded as Y shaped containing two Heavy Chains (HC) and two Light Chains (LC). mAbs are currently produced through a bioprocess that involved recombinant DNA technology, cell transfection, selection of clones and finally expansion of the best-producer clone within large-scale bioreactor to accumulate the mAbs secreted in the culture medium. The post-translational modifications like glycosylation are necessary for proper folding, oligomerization, stability, tissue targeting and biological function of the mAbs. Currently, the main manufacturers (Abbott/Abbvie, Sanofi, Merck, Johnson and Johnson, Bristol-Myers Squibb, Amgen, Regeneron Pharmaceuticals Inc., Novartis, Genentech/Roche, Pfizer, AstraZeneca....) are producing mAbs using mammalian cell lines as bio-factories, like for example Chinese Hamster Ovary (CHO) cell line. This is because of its growth behavior, its ability to excrete proteins in significant amounts and its human-like glycosylation.

However, after more than 40 years of optimization, production in CHO cells remains imperfect. For example, mAb produced in CHO cells are heterogeneous. Indeed, they do not have a homogenous glycosylation pattern but rather a mixture of glycoforms, which is not easily controlled and can vary between batches. Thus, metabolic engineering strategies have still to be implemented in CHO cells. Moreover, mAbs produced in CHO cells are subjected to proteolytic degradations, thus increasing the heterogeneity of the product. In addition, CHO cell expression system present other disadvantages like contamination risk with viruses and prions that could be harmful to humans, thus requiring numerous steps of purification and quality control. In routine manufacturing at a 10,000 L scale, culture media, complex nutritional requirement and fermentation as well as downstream processing steps contribute to the high manufacturing costs of mAbs using mammalian cells as bio-factories. Finally, mAbs produced in such conditions are very expensive.

Due to these limitations, there is interest to develop alternative expression systems for the production of mAbs in order to decrease their production costs, making them more accessible.

Alternative expression systems like plants have emerged. However, the production of biopharmaceuticals in tobacco plants require specific hands-on to transform and grow plants efficiently and require to build entirely new infrastructures and production unit including greenhouses.

Microalgae are photosynthetic organisms like plants while being unicellular microorganisms that can be grown as mammalian cells in self-contained systems like Petri dishes, flasks or bioreactors requiring cell culture expertise. Culture media is estimated to be much lower (0.002 US$ per liter) that is especially true for photosynthetic microalgae for which sunlight drives the production of biomass (Rosales- Mendoza et al., 2020). Furthermore, microalgae are less susceptible to virus harmful to humans (Yolken et al., 2014). In addition, significant improvement in genome-editing tools led recently to consider microalgae as alternative cell bio-factory for the production of recombinant proteins (Rosales-Mendoza et al., 2020).

Diatoms like *Phaeodactylum tricornutum* (later abbreviated as *P. tricornutum* or PHAEO) have been proposed to be used for the production of recombinant proteins. They are unicellular eukaryotic cells responsible for about 20% of the global carbon dioxide fixation and for biogeochemical cycling of important nutrients. *P. tricornutum* is believed to have arisen via a serial secondary endosymbiotic event in which a green alga and subsequently a red one were engulfed by a heterotrophic eukaryote, thus generating specific genomic characteristics. Indeed, a recent investigation of *P. tricornutum* genome revealed that 3,170 genes (26%) are unique and specific to this organism (Rastogi et al., 2018). *P. tricornutum* also harbors a combination of metabolic pathways that belongs to either the plant or animal kingdoms (Butler et al., 2020). This represents unique biological features, responsible for the great adaptability of *P. tricornutum* within the environment (Tirichine and Bowler, 2011) and its capacity to grow in different culture conditions such as hyposaline conditions, low temperature or low light (Gutenbrunner et al., 1994; Bartual et al., 2008; De Martino et al., 2007; De Martino et al., 2011). Thus, the flexible and unique nature of *P*. *tricornutum* offers immense possibilities to produce sustainable recombinant proteins.

*P. tricornutum* naturally synthesizes numerous compounds of interest like pigments, carotenoids, EPA and omega-3 fatty acids (Butler et al., 2020). Transformation methods and development of molecular tools, especially genetic engineering such as gene silencing, TALEN and Crisper/cas9, pave the way for biotechnological applications like the production of recombinant therapeutic proteins in *P. tricornutum* (Rosales-Mendoza et al., 2020). Indeed, *P. tricornutum* has been used to produce recombinant human anti-Marburg virus mAbs (Hempel et al., 2017) and functionally glycosylated human anti-hepatitis B mAbs (Hempel et al., 2011; Hempel and Maier, 2012; Vanier et al., 2015 and 2018). The quality of these anti-Hepatitis B mAb was confirmed using a Multi-Attribute Monitoring (MAM) approach, thus demonstrating that the production of mAbs in *P. tricornutum* was homogeneous and of good-quality. It was shown that the C-terminal ends of the heavy chains of the *P. tricornutum* -made mAbs remain intact in contrast to what is usually observed in mAbs produced in CHO cells. In addition, cleavage of the human signal peptide from the anti-Hepatitis B mAb produced in *P. tricornutum* was efficient, thus confirming that the protein secretion in *P. tricornutum* occurs as reported in mammals (Vanier et al., 2015). With regards to its biological activity, it was demonstrated that the *P. tricornutum* -made mAb is able to bind human Fcy receptors including FcyRI and FcγRIIIa, which suggests that it could be used efficiently in human immunotherapy to induce phagocytosis and antibody-dependent cell mediated cytotoxicity (ADCC) response (Vanier et al., 2018). In addition, it was demonstrated that *P. tricornutum* is able to glycosylate the Fc parts of mAbs with oligomannosides bearing 5 to 9 mannose residues (Vanier et al., 2015). These oligomannosides are structurally identical to those of mammals (Dumontier et al., 2021), which contrast to other microalgae like the green microalgae *Chlamydomonas reinhardtii* and *Chlorella vulgaris* (Toustou et al., 2022). Thus, *P. tricornutum* is able to produce and secrete mAbs exhibiting a human-like glycosylation.

Even successful, industrial exploitation is still limited due to the low amount of mAbs secreted within the culture medium. Thus, increasing the production yield is the bottleneck to unlock before any industrialization of *P. tricornutum* as a green eco-friendly expression system for the production of mAbs can be envision.

There is therefore a need to identify innovative strategies to increase the secretion and production yields of monoclonal antibodies in *P. tricornutum.*

The present inventors solve this need by proposing a unique improved *P. tricornutum* nucleotide construct encoding therapeutic monoclonal antibodies (for example Trastuzumab and Rituximab), and recombinant *P. tricornutum* strains containing said construct, said cells exhibiting an higher secretion and mAbs production yield while being robust at the industrial scale.

The example studies below have been carried out on two anti-cancer mAbs namely Trastuzumab and Rituximab. Both mAbs belong to the 21st WHO Model List of Essential Medicines (World Health Organization, 2019) and are used to treat cancers that in 2018 caused 9.6 million deaths with cancer worldwide incidence increasing to 18.1 million cases, representing multibillion dollar industries.

### Description of the invention

In order to increase the production yield and secretion of these mAbs in *P. tricornutum's* culture medium, different promoter/terminator couples were tested. It is herein demonstrated that the V-ATPase C promoter/FcpA terminator pair can increase the yield of mAbs production in *P*. *tricornutum* in an 8-day bioprocess. It has also been highlighted that the use of the FcpA terminator is preferential to the endogenous terminator associated with the promoter for an increase in production yield. In parallel, the influence of different heterologous and endogenous signal peptides was tested on the secretion of antibodies in the culture medium. Surprisingly, the heterologous signal peptide "E" was shown to be more effective than the endogenous one of the HASP1 protein that is the most abundant secreted protein in *P. tricornutum's* culture medium. These results contribute to the characterisation of various key elements to be considered in the vector design in order to increase the production yield of recombinant proteins in *P. tricornutum.*

Moreover, the selection of a strong promoter that drives high yields of mAbs is a prerequisite for developing a cost-effective production platform. To date, only few endogenous promoters have been characterized in *P. tricornutum.* In the present work, transcriptomic data from *P*. *tricornutum* Pt3 strain were analysed and thirty-three potential "strong" endogenous promoters were identified. First, these putative promoter sequences were cloned and fused to the coding sequence of the enhanced green fluorescent protein (eGFP). Their strengths were assessed by measuring eGFP fluorescence, thus reflecting the level of eGFP expression. Among the thirty-three promoters, thirteen were able to drive eGFP expression. Best results were obtained with the VOC promoter (Phatr3_J34085, SEQ ID NO:33) which allowed a significant increase expression of eGFP by comparison to the one obtained with the inducible NR promoter. These findings contribute to the discovery of new genetic tools that could be used to improve recombinant protein production in *P. tricornutum.*

In the present studies, two antibodies were chosen as proof of concepts. The first one is a therapeutic mAb used to target human epidermal growth factor receptor 2 involved in HER2-positive breast and gastric cancers, named Trastuzumab. The second one is the Rituximab, an anti-CD20 recombinant mAb that has been commercialized to treat lymphoma. Its clinical use has now been extended to treat auto immune diseases.

Altogether, the present invention provides a new system for producing high amounts of therapeutic antibodies, functional fragments or derivatives thereof, in microalgae. Said therapeutic antibodies, functional fragments or derivatives thereof are secreted in the extracellular medium of said microalgae, from which they can easily be purified.

In a first aspect, the present invention relates to a single polynucleotide vector expressing a therapeutic monoclonal antibody or a functional fragment or derivative thereof, said vector sequence comprising:
a) nucleotide sequences encoding, in the same or in two different Open Reading Frame(s):
   - the light chain of said therapeutic antibody, fragment or derivative thereof, preceded at its *N*-terminal end by a first heterologous secretion signal peptide,
   - the heavy chain of said therapeutic antibody, fragment or derivative thereof, preceded at its N-terminal end by a second heterologous secretion signal peptide,
   said light and heavy chain being separated by a nucleotide sequence encoding a linker,
b) at least one promoter operatively linked with the sequences encoding said light and heavy chains, said promoter being able to drive the expression of said light and heavy chains in the microalgae *Phaeodactylum tricornutum,*
c) at least one translation termination sequence, and
d) optionally, at least one detectable marker.

All the important features of the vector of the invention will now be described one by one.

### Secretion signal peptides useful in the vector of the invention

The polynucleotide vector of the invention encodes at least two heterologous secretion signal peptides, said heterologous signal peptides enabling the secretion of the two different polypeptide chains of the therapeutic antibody, of the functional fragment or of the derivative thereof, into the extracellular environment, herein being the culture medium.

Preferably, these heterologous secretion signal peptides are located at the *N*-terminal end of each polypeptide chains, whose secretion is therefore independently regulated. These secretion signal peptides are typically 15-30 amino acids long.

In particular, the polynucleotide of the invention encodes:
- the light chain of said therapeutic antibody, fragment or derivative thereof, preceded at its *N*-terminal end by a first heterologous secretion signal peptide,
- the heavy chain of said therapeutic antibody, fragment or derivative thereof, preceded at its N-terminal end by a second heterologous secretion signal peptide.

In one embodiment, these first and second heterologous secretion signal peptides are identical.

In another embodiment, these first and second heterologous secretion signal peptides are different.

In the context of the invention, the secretion signal peptides can be chosen in the group consisting of:
- Signal peptide E of azurocidine disclosed in Kober et al 2013 (SEQ ID NO:1)
- Signal peptide B disclosed in Kober et al 2013 (SEQ ID NO:5)
- Signal peptide B from Human Serum Albumin modified as described in Attalah et al., 2017 (SEQ ID NO:85)
- Signal peptides disclosed in Table 1 of Kober et al 2013
- Signal peptide H5 disclosed in Haryadi 2015 (SEQ ID NO:2)
- Signal peptide H7 disclosed in Haryadi 2015 (SEQ ID NO:3)
- Signal peptide L1 disclosed in Haryadi 2015 (SEQ ID NO:4)
- Signal peptide H1, H2, H3, H4, H6, H8 and L2 by reference to Table 1 of Haryadi et al. 2015
- Signal peptide of the human BIP protein (SEQ ID NO:6)
- Signal peptide of the BIP protein from *Phaeodactylum tricornutum* (SEQ ID NO:7 or SEQ ID NO:8)
- Signal peptide of the human PDI protein (SEQ ID NO:9)
- Signal peptide of the PDI protein from *Phaeodactylum tricornutum* (SEQ ID NO:82)
- Signal peptide used in Hempel and Maier 2012 which is incorporated by reference
- Signal peptide used in Hempel et al., 2017 which is incorporated by reference
- Signal peptide of gametolysin, disclosed in Ramos-Martinez et al., 2017
- The signal peptides found in proteins that are naturally secreted by *Phaeodactylum tricornutum,* identified recently in Chuberre et al, 2022, which is incorporated by reference.

In a preferred embodiment, the polynucleotide vector of the invention contains at least one secretion signal peptide chosen in the group consisting of: E of SEQ ID NO:1, H5 of SEQ IDNO:2, H7 of SEQ ID NO:3 or L1 of SEQ ID NO:4.

In a preferred embodiment, said first and second heterologous secretion signal peptides are L1 of SEQ ID NO:4 (preceding the sequence of the light chain) and H7 of SEQ ID NO:3 (preceding the sequence of the heavy chain) respectively.

In a preferred embodiment, said first and second heterologous secretion signal peptides are identical, and are more preferably the E signal peptide of SEQ ID NO:1 (in other terms, both chains are regulated by the same signal peptide, which is E).

In a preferred embodiment, said first and second heterologous secretion signal peptides are followed by or include a sequence encoding a cleavage site, so that the signal peptides can be removed in the endoplasmic reticulum once their function has been fulfilled.

These signal peptides can be encoded by codon optimized sequences that are easily identifiable by the skilled person. For example, it is possible to use in the vectors of the invention any of:
- SEQ ID NO:86 (Codon-optimized DNA sequence coding for signal peptide "E")
- SEQ ID NO:87 (Codon-optimized DNA sequence coding for signal peptide "H5")
- SEQ ID NO:88 (Codon-optimized DNA sequence coding for signal peptide "H7")
- SEQ ID NO:89 (Codon-optimized DNA sequence coding for signal peptide "L1")
- SEQ ID NO:90 (Codon-optimized DNA sequence coding for signal peptide of the human BIP)
- SEQ ID NO:91 (Codon-optimized DNA sequence coding for signal peptide of the human PDI).

### Promoters useful in the vector of the invention

Promoters can be incorporated into a vector using standard techniques known in the art. In one embodiment, the promoter can be positioned at about the same distance from the transcription start site as it is from the transcription start site in its natural genetic environment. Some variation in this distance is permitted without substantial decrease in promoter activity. A transcription start site is typically included in the vector.

Exemplary promoters that can be used in the vector of the invention in order to drive the expression of the encoded polypeptides include, but are not restricted to, promoters such as:
- The promoter of the *H4-1B* gene disclosed in Slattery et al, 2018 (SEQ ID NO:24)
- The promoter of the *V-ATPase* C gene disclosed in Watanabe et al, 2018 (SEQ ID NO:25)
- The endogenous promoter of the NR gene of *Phaeodactylum tricornutum* disclosed in Hempel et al., 2009 and in Stork et al., 2012 (SEQ ID NO:26)
- The endogenous promoter of the *fcpA*/*lhcf1* gene of *Phaeodactylum tricornutum* (disclosed in Bhaya and Grossman, 1993; Apt et al., 1995; Apt, Kroth-Pancic and Grossman, 1996; Falciatore et al., 1999) (SEQ ID NO:27)
- The endogenous promoter of the *fcpb* /*lhcf2* gene of *Phaeodactylum tricornutum* (disclosed in Bhaya and Grossman, 1993; Apt et al., 1995; Apt, Kroth-Pancic and Grossman, 1996; Falciatore et al., 1999) (SEQ ID NO:28)
- The promoter HASP1 as described in Erdene-Ochir et al., 2019 (SEQ ID NO:83)
- The promoter 40SRPS8 as described in Slattery et al, 2018 (SEQ ID NO:84)

It is also possible to use any of the following promoters, which have been identified as they are associated to genes that are upregulated in *Phaeodactylum tricornutum* being subjected to saline stress (culture in 10% sea water):
- Promoter of the *Phatr3_J37038* gene (SEQ ID NO:29)
- Promoter of the *Phatr3_Jdraft1443* gene (SEQ ID NO:30)
- Promoter of the *Phatr3_J35102* gene (SEQ ID NO:31)
- Promoter of the *Phatr3_J50252* gene (SEQ ID NO:32)
- Promoter of the *Phatr3_J34085* gene (SEQ ID NO:33)
- Promoter of the *Phatr3_J48164* gene (SEQ ID NO:34)
- Promoter of the *Phatr3_EG02422* gene (SEQ ID NO:35)
- Promoter of the *Phatr3_J42538* gene (SEQ ID NO:36)
- Promoter of the *Phatr3_J48356* gene (SEQ ID NO:37)

Multiple copies of promoters or multiple promoters can be used in the vectors of the invention. For example, it is possible to associate parts of the above-disclosed promoters to construct chimeric promoter sequences that are as efficient as (or more efficient than) the original promoters.

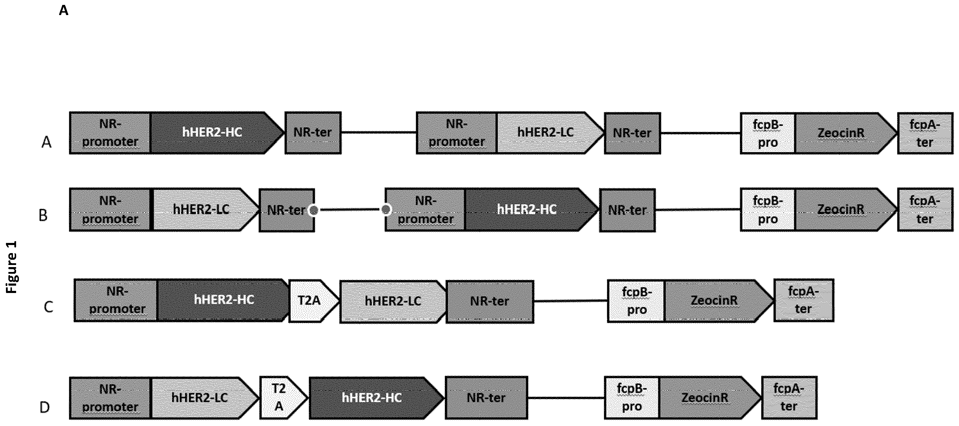

In particular, it is possible to use vectors in which the coding sequence of each chain of the therapeutic antibody is operatively linked to a different promoter (figure 1B). In a particularly preferred case, the vector of the invention can contain an origin of replication (ORI) between the two sequences encoding each antibody chain (see for example the schemes of the vectors E) to J) on figure 1B).

It is possible to use vectors in which the two coding sequences are positioned in the same direction for translation (in this case, all the coding sequences can be located within the same Open Reading Frame, see vectors E) and H) of figure 1B), or in opposite direction for translation (in this case, the vector shall contain two Open Reading Frames). The vectors F, G, I and J proposed on figure 1B typically display the two coding sequences in opposite directions.

The two promoters are preferably chosen in the group consisting of: the promoter of the *Nitrate Reductase* gene from *Phaeodactylum tricornutum* (SEQ ID NO:26), the promoter of the *FcpA* gene from *Phaeodactylum tricornutum* (SEQ ID NO: :27), the promoter of the *FcpB* gene from *Phaeodactylum tricornutum* (SEQ ID NO:28), the promoter of the *V-ATPase-C* gene (SEQ ID NO:25), the promoter of the *histone H4-1B* gene (SEQ ID NO24), the promoter of the *Phatr3_J37038* gene (SEQ ID NO:29), the promoter of the *Phatr3_Jdraft1443* gene (SEQ ID NO:30), the promoter of the *Phatr3_J35102* gene (SEQ ID NO:31), the promoter of the *Phatr3_J50252* gene (SEQ ID NO:32), the promoter of the *Phatr3_J34085* gene (SEQ ID NO:33), the promoter of the *Phatr3_J48164* gene (SEQ ID NO:34), the promoter of the *Phatr3_EG02422* gene (SEQ ID NO:35), the promoter of the *Phatr3_J42538* gene (SEQ ID NO:36), and the promoter of the *Phatr3_J48356* gene (SEQ ID NO:37).

### Self-cleaving linkers used in the vector of the invention

When the two polypeptide chains are within the same ORF, they should be separated by a self-cleaving "linker" sequence ensuring that the two chains of the same ORF are appropriately cleaved after their translation in the microalgae cells. This self-cleaving peptide is for example a 2A linker peptide, that is, a 18-22 amino acid long peptide which can induce ribosomal skipping during translation of the antibody chains in the microalgae cells. This linker peptide and others are described for example in Szymczak et al., 2004.

In a preferred embodiment, said self-cleaving linker sequence is chosen in the group consisting of:
- Self-cleaving linker peptide E2A (SEQ ID NO:10)
- Self-cleaving linker peptide F2A (SEQ ID NO:11)
- Self-cleaving linker peptide variant of F2A (SEQ ID NO:12) from Zou et al 2018 or from Ho et al, 2013 (SEQ ID NO:13)
- Self-cleaving linker peptide P2A (SEQ ID NO:14)
- Self-cleaving linker peptide T2A (SEQ ID NO:15)

It is also possible to use any known means to induce appropriate cleavage, and in particular the Internal Ribosome Entry Site (IRES) elements that have successfully been used to induce monoclonal antibody expression in CHO cells (Ho et al., 2012, 2013 and Chng et al., 2015). Useful IRES linkers have for example the SEQ ID NO:16 and SEQ ID NO:17.

In a particular embodiment, it is advantageous to add a furin cleavage sequence upstream of the 2A linker peptide to eliminate two amino acids that would otherwise remain attached to the upstream antibody chain after cleavage. This furin-cleavage site has for example the sequence RRKR (SEQ ID NO:18). As disclosed in Chng et al., 2015, it would be also advantageous to add a GSG linker between the furin-cleavage site and the 2A linker, in order to enhance the cleavage efficiency and the expression of the antibodies. Repeated GSG sequences can be also used as preferred linkers.

### Translation termination sequences useful in the vector of the invention

The polynucleotide vector of the invention may contain other regulatory components that are functional in the microalgae cells in which the vector is to be expressed. Regulatory elements include, for example, 5' promoters, transcription factor binding sites, Ubiquitous Chromatin-Opening Elements, InRNAs, Kozak, transcription termination sequences, translation termination sequences, enhancers, and polyadenylation elements.

Translation termination are typically positioned downstream of a coding sequence to provide for efficient termination of the translation into the encoded polypeptide.

In the vector of the invention, it is possible to use, in particular, the following translation termination sequences:
- Termination sequence of the *FcpA* gene as disclosed in Apt et al 1996 (SEQ ID NO:19)
- Termination sequence of the NR gene as disclosed in Hempel et al., 2009 and Stork et al., 2012 (SEQ ID NO:20)
- Termination sequence of the *H4-1B* gene as disclosed in Slattery et al 2018 (SEQ ID NO:21)
- Termination sequence of the *V-ATPase* C gene (SEQ ID NO:22)
- Termination sequence of the *γ-tubulin* gene as disclosed in Slattery et al 2018 (SEQ ID NO:23)

It is also possible to use other termination sequences, such as those cited in Slattery et al., 2018, notably the termination sequences of the *EF1-α* gene, of the *40SRPS8* gene, of the *RBCMT* gene, of the *fcpB, fcpC, fcpD,* or *fcpF* genes.

It is also possible to use other termination sequences, such as those cited in Windhagauer et al., 2021, notably the termination sequences of the *NADH:Ubiquinone oxidoreductase* gene, of the *SynaptobrevinNAMP-like protein* gene, of the *Phatr3_J45582* gene, or of the *Prohobitin* gene.

Finally, it is also possible to use the termination sequences of the genes whose promoters are recited in SEQ ID NO:29-37, namely the termination sequence of Phatr3_J37038 (SEQ ID NO:59), of Phatr3_Jdraft1443 (SEQ ID NO:60), of Phatr3_J35102 (SEQ ID NO:61), of Phatr3_J50252 (SEQ ID NO:62), of Phatr3_J34085 (SEQ ID NO:63), of Phatr3_J48164 (SEQ ID NO:64), of Phatr3_EG02422 (SEQ ID NO:65), of Phatr3_J42538 (SEQ ID NO:66), or of Phatr3_J48356 (SEQ ID NO:67).

In a preferred embodiment, the vector of the invention contains a translation termination sequence chosen among : the termination sequence of the *Nitrate Reductase* gene (SEQ ID NO:20) or the termination sequence of the *FcpA* gene of *Phaeodactylum tricornutum* (SEQ ID NO:19), the termination sequence of the *V-ATPase-C* gene (SEQ ID NO:22), the termination sequence of the *γ-tubulin* gene (SEQ ID NO:23), the termination sequence of the *histone H4* gene (SEQ ID NO:21) and the termination sequence of the of Phatr3_J34085 gene (SEQ ID NO:63).

### Coding sequences for therapeutic molecules

The vector of the invention can encode any monoclonal therapeutic antibody.

In particular, it can encode any chimeric, humanized, or human monoclonal antibody, said antibody having anti-viral, anti-auto-immune or anti-cancer therapeutic action on human or on animal subjects.

In a particular embodiment, said antibody can be chosen in the group consisting of: rituximab, trastuzumab, adalimumab, bevacizumab, infliximab, cetuximab, motavizumab, palivizumab, alemtuzumab, dinutuximab, naxitamab but also comprising for instance, benralizumab, catumaxomab, daratumumab, elotuzumab, epratuzumab, farletuzumab, galiximab, gemtuzumab ozogamicin, ibritumomab tiuxetan, lumiliximab, necitumumab, nimotuzumab, ocrelizumab, ofatumumab, oregovomab, pertuzumab, tositumomab, zalutumumab, and zanolimumab. Any other useful antibodies can be used (see Walsh et al, 2018).

The polypeptide sequences of the constant and variable chains of all these antibodies are well known, as they have been published and well described. They do not need to be detailed here.

In a preferred embodiment, the vector of the invention encodes either the Trastuzumab (Herceptin) antibody or the Rituximab antibody or the dinutuximab antibody or the naxitamab antibody or any functional variant thereof.

For producing the antibody TrastuzumAb (Herceptin), the vector of the invention can contain the light chain of SEQ ID NO:38 and the heavy chain of SEQ ID NO:39 (chains without signal peptides).

For producing the antibody RituximAb, the vector of the invention can contain the light chain of SEQ ID NO:40 and the heavy chain of SEQ ID NO:41 (chains without signal peptides).

### Particular vectors of the invention

In a preferred embodiment, the vector of the invention contains:
said promoter is the promoter of the *Nitrate Reductase* gene of SEQ ID NO:26 or the promoter of the *V-ATPase-C* gene of SEQ ID NO:25 or the promoter of the *Phatr3_J34085* gene of SEQ ID NO:33 and
- the termination sequence of the *FcpA* gene of *Phaeodactylum tricornutum* of SEQ ID NO:19.

In a more preferred embodiment, the vector of the invention contains, in this order:
- the promoter of *V-ATPase* C gene of SEQ ID NO:25 or the promoter *Phatr3_J34085* gene (SEQ ID NO:33),
- the sequence encoding the secretion peptide signal E of SEQ ID NO:1,
- the sequence encoding the light chain of said therapeutic antibody, fragment or derivative thereof,
- the sequence encoding the linker T2A of SEQ ID NO:15 or others variants,
- the sequence encoding the secretion peptide signal E of SEQ ID NO:1,
- the sequence encoding the heavy chain of said therapeutic antibody, fragment or derivative thereof,
- the termination sequence of the *FcpA* gene of *Phaeodactylum tricornutum* of SEQ ID NO:19.

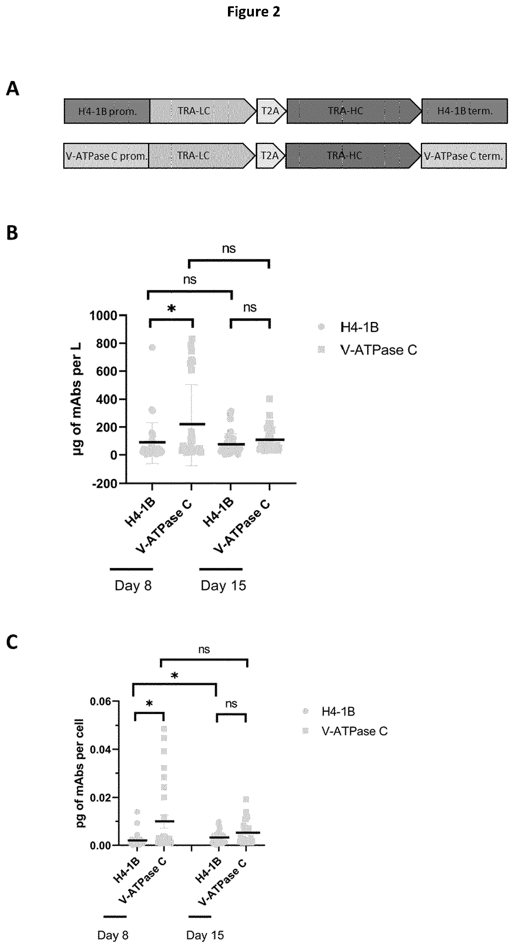

The following vectors have been successfully generated by the inventors for producing Herceptin (TrastuzumAb). As shown in the examples below (and figures 2-4), they are able to produce high yields of said monoclonal antibody in *Phaeodactylum tricornutum* cells of different strains.

| | | |
|---|---|---|
| Vector NVB5 | promoter of NR- LC of herceptin-T2A-HC of herceptin -fcpA terminator | SEQ ID NO:42 |
| Vector NVB6 | promoter of H4-1B- LC of herceptin -T2A-HC of herceptin - pH4-1b terminator | SEQ ID NO:43 |
| Vector NVB7 | promoter of V-ATPase-C- LC of herceptin -T2A-HC of herceptin - V-ATPase-C terminator | SEQ ID NO:44 |
| Vector C | promoter NR-HC of herceptin - T2A-LC of herceptin -NR terminator | SEQ ID NO:45 |
| Vector D | promoter NR-LC of herceptin - T2A-HC of herceptin -NR terminator | SEQ ID NO:46 |
| Vector Pro1D | Promoter 37038 + LC of herceptin -T2A-HC of herceptin - NR terminator | SEQ ID NO:47 |
| Vector Pro2D | Promoter D1443 + LC of herceptin -T2A-HC of herceptin - NR terminator | SEQ ID NO:48 |
| Vector Pro3D | Promoter 35102 + LC of herceptin -T2A-HC of herceptin - NR terminator | SEQ ID NO:49 |
| Vector Pro4D | Promoter 50252 + LC of herceptin -T2A-HC of herceptin - NR terminator | SEQ ID NO:50 |
| Vector Pro5D | Promoter 34085 + LC of herceptin -T2A-HC of herceptin - NR terminator | SEQ ID NO:51 |
| Vector Pro6D | Promoter 48164 + LC of herceptin -T2A-HC of herceptin - NR terminator | SEQ ID NO:52 |
| Vector Pro7D | Promoter EG02422 + LC of herceptin -T2A-HC of herceptin - NR terminator | SEQ ID NO:53 |
| Vector Pro7D+ Kozak | Promoter EG02422+Kozak sequence + LC herceptin -T2A-HC herceptin - NR terminator | SEQ ID NO:54 |

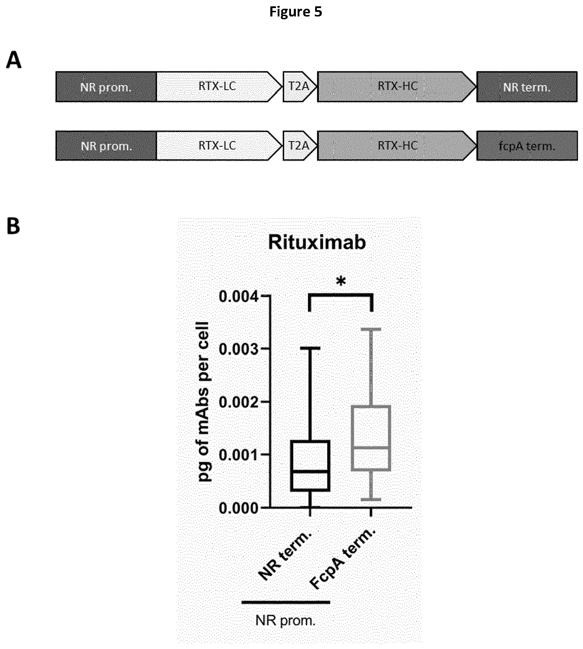

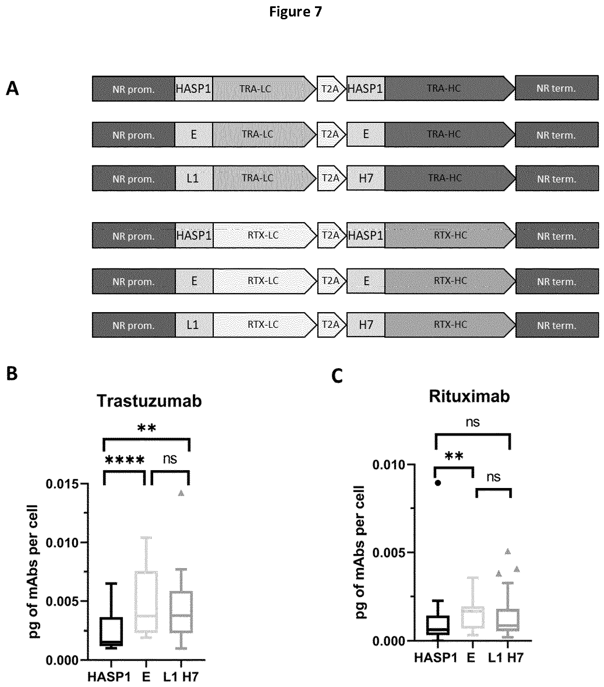

The following vectors have been successfully generated by the inventors for producing RituximAb. As shown in the examples below (NVB12, NVB13 and NVB16 on figures 5 and 7), they are able to produce high yields of said monoclonal antibody in *Phaeodactylum tricornutum* cells of different strains (Pt1 to 10, Rastogi et al, 2019).

| | | |
|---|---|---|
| Vector NVB12 | promoter of NR- LC of RituximAb -T2A-HC of RituximAb -NR terminator | SEQ ID NO:55 |
| Vector NVB13 | promoter of NR- LC of RituximAb -T2A-HC of RituximAb - fcpA terminator | SEQ ID NO:56 |
| Vector NVB14 | promoter of NR- L1-LC of RituximAb -T2A-H7-HC of RituximAb - NR terminator | SEQ ID NO:57 |
| Vector NVB16 | promoter of NR- E-LC of RituximAb -T2A-E-HC of RituximAb - NR terminator | SEQ ID NO:58 |

### Recombinant cells of the invention

A second aspect of the invention concerns transformed or recombinant microalgae cells containing the polynucleotide vector of the invention, as disclosed above.

Said microalgae cells according to the invention are selected from the genus *Tetraselmis, Porphyridium, Symbiodinium, Thalassiosira, Nannochloropsis, Emiliania, Pavlova, Isochrysis, Eutreptiella, Euglena, Phaeodactylum, Dunaliella, Chlorella, Haematococcus,* and *Scenedesmus.*

In a preferred embodiment, said microalgae cells according to the invention are selected among the species *Phaeodactylum tricornutum, Nannochloropsis oculate, Isochrysis galbana, Tetraselmis suecica, Dunaliella salina, Chlorella, Scenedesmus,* and *Hematococcus.*

In an even more preferred embodiment, said microalgae cells correspond to *Phaeodactylum tricornutum,* and are from the strains Pt1, Pt2, Pt3, Pt4, Pt5, Pt6, Pt7, Pt8, Pt9 or Pt10.

In a most preferred embodiment, said microalgae cells are transformed cells of *Phaeodactylum tricornutum* from the strains Pt1, Pt3, Pt4, Pt5 and Pt7. The inventors indeed demonstrated that the vectors of the invention produce high levels of monoclonal antibodies from Pt1 and Pt3 cells (example 2).

Transformation of the microalgae cells of the invention can be carried out by conventional methods such as microparticles bombardment (Biolistic), electroporation, bacterial conjugation leading to episomal expression.

In an embodiment of the invention, nucleotide sequences may be introduced into the microalgae cells of the present invention via a plasmid, virus sequences, double or simple strand DNA, circular or linear DNA.

It is generally desirable to include into each nucleotide sequences or vectors at least one selectable marker to allow the selection of the microalgae cells that have been stably transformed. Examples of such markers are antibiotic resistant genes such as *sh ble* gene enabling resistance to zeocin, *nat* or *sat-1* genes enabling resistance to nourseothricin, *aph8* enabling resistance to paromomycin, *nptll* enabling resistance to G418. The use of auxotrophic strains of *P*. *tricornutum* can also be used for the selection of microalgae that have been transformed (both stably or transiently). Auxotrophic strains for uracil, tryptophan and histidine have been generated as described in literature, but auxotrophy to leucin or methionine can also be considered. The generation of strains with particular genes inactivated can also allow positive selection of transformed strains. For example, inactivation of the *umps* and *apt* genes allows resistance to 5-FOA (5-Fluoroorotic acid) and 2-FA (2-fluoroadenine) respectively.

After transformation of microalgae of the present invention, transformants producing the desired therapeutic antibodies, functional fragments or derivatives thereof secreted in the culture media are selected. Selection can be carried out by one or more conventional methods comprising: enzyme-linked immunosorbent assay (ELISA), mass spectroscopy such as MALDI-TOF-MS, ESI-MS chromatography, spectrophotometer, fluorimeter, immunocytochemistry by exposing cells to an antibody having a specific affinity for the desired therapeutic antibodies, functional fragments or derivatives thereof.

### Optimized methods to produce therapeutic molecules using the vectors of the invention

In another aspect, the invention relates to a method for producing a therapeutic antibody, a functional fragment or a derivative thereof which is secreted in the extracellular medium, said method comprising the steps of:
(i) culturing the transformed microalgae cells of the present invention as described here above;
(ii) harvesting the extracellular medium of said culture; and
(iii) purifying the therapeutic antibody, a functional fragment or a derivative thereof, which is secreted in said extracellular medium.

The method of claim 13, wherein said appropriate culture conditions involve the use of a culture medium containing a nitrate concentration comprised between 0,1g/L and 1g/L and low salt concentration comprised between 5 to 30%.

In a particular example, the strain of *P*. *tricornutum* can be cultivated between 15°C and 25°C, preferably at 19°C. It can be cultivated for example on a photoperiod of 16/ 8h light/ dark cycle with light intensity of 30 µmol photons.m⁻².s⁻¹. It can be cultivated either on agar plates or in liquid cultures under agitation at 150 rpm. The cells can be grown in 100 % artificial seawater (33.3 g.L⁻¹ of sea salt (Instant Ocean^{®}, Aquarium System, Sarrebourg, France)) filtered through 0.45 µm filters and sterilized by autoclave. The culture can last 15 days, preferentially between 3 and 10 days, more preferentially 8 days.

They are preferably cultivated in a sterilized culture medium containing between 5% to 30% sea water. This medium should be supplemented with 1 mL.L⁻¹ of Conway NaNO₃ nutritive medium (said nutritive medium containing Na₂EDTA.2H₂O : 45g.L⁻¹; NaNO₃ : 100 g.L⁻¹; H₃BO₃ : 33,6 g.L⁻¹; NaH₂PO₄ : 20 g.L⁻¹; FeCl₃: 0,768 g.L⁻¹; ZnCl₂: 21 mg.L⁻¹; CoCl₂, 6H₂O : 20 mg.L⁻¹; (NH₄)₆Mo₇O₂₄, 4H₂0 : 9 mg.L⁻¹; CuSO₄, 5H₂O : 20 mg.L⁻¹; MnCl₂,4H₂O : 360 mg.L⁻¹; B₁ vitamin : 200 mg.L⁻¹; B₁₂ vitamin : 10 mg.L⁻¹). Optionally 80 mg.L⁻¹ of sodium metasilicate (Na₂SiO₃) can be added.

In a preferred embodiment, the Man and Meyers medium (M&M medium) is used to cultivate the strains of *P*. *tricornutum.*

The composition of the M & M medium is given below:

| | Mann and Myers' medium | |
|---|---|---|
| | Component | Concentration |
| Salinity | NaCl | 5 g/L |
| | MgSO₄·7H₂O | 1.2 g/L |
| | KCI | 0.6 g/L |
| | CaCl₂ | 0.3 g/L |
| Nitrate | NaNO₃ | 11.76 mM |
| Phosphate | K₂HPO₄ | 0.574 mM |
| Tris | Tris | 1.0 g/L |
| Trace element | Na₂EDTA | 8.92 × 10⁻⁵ M |
| | H₃BO₃ | 9.70 × 10⁻⁵ M |
| | FeSO₄·7H₂O | 7.19 × 10⁻⁶ M |
| | MnCl₂ | 1.11 × 10⁻⁵ M |
| | ZnSO₄·7H₂O | 1.15 × 10⁻⁶ M |
| | Co(NO₃)₂·6H₂O | 2.40 × 10⁻⁸ M |
| | CuSO₄·5H₂O | 8.01 × 10⁻⁹ M |

### Definitions

The term "polynucleotide" used herein refers to DNA molecules (e.g., cDNA or genomic or synthetic DNA) and RNA molecules (e. g., mRNA or synthetic RNA), as well as analogs of DNA or RNA containing non-natural nucleotide analogs, non-native internucleoside bonds, or both. Preferably, said polynucleotide is a DNA molecule. The polynucleotide can be in any topological conformation, like linear or circular.

"Operatively linked" promoter refers to a linkage in which the promoter is contiguous with the gene of interest to control the expression of said gene.

The term "signal peptide" as used herein refers to an amino acid sequence which is generally located at the amino terminal end of the amino acid sequence of a therapeutic antibody or functional fragment thereof. The signal peptide mediates the translocation of said therapeutic antibody, functional fragment or derivative thereof through the secretion pathway and leads to the secretion of said therapeutic antibody, functional fragment or derivative thereof in the extracellular medium.

As used herein, the term "secretion pathway" refers to the process used by a cell to secrete proteins out of the intracellular compartment. Such pathway comprises a step of translocation of a polypeptide across the endoplasmic reticulum membrane, followed by the transport of the polypeptide in the Golgi apparatus, said polypeptide being subsequently released in the extracellular medium of the cell by secretory vesicles. Post-translational modifications necessary to obtain mature proteins, such as glycosylation or disulfide bonds formation, are operated on proteins during said secretion pathway.

The term "heterologous", with reference to a signal peptide according to the invention, means an amino acid sequence which does not exist in the corresponding microalga before its transformation, and preferably originate from another organism (not a microalga). It is intended that the term encompasses proteins that are encoded by wild-type genes, mutated genes, and/or synthetic genes.

The term "antibody" is used herein in the broadest sense and specifically covers monoclonal antibodies (including full length monoclonal antibodies) of any isotype such as IgG, IgM, IgA, IgD, and IgE, polyclonal antibodies, multispecific antibodies, chimeric antibodies, and antigen-binding fragments. An antibody reactive with a specific antigen can be generated by recombinant methods such as selection of libraries of recombinant antibodies in phage or similar vectors, or by immunizing an animal with the antigen or an antigen-encoding nucleic acid.

In a preferred embodiment, the antibodies of the invention are monoclonal antibodies. As used herein, the term "monoclonal antibody" refers to an antibody arising from a nearly homogeneous antibody population. More particularly, the individual antibodies of a population are identical except for a few possible naturally-occurring mutations which can be found in minimal proportions. In other words, a monoclonal antibody consists of a homogeneous antibody population arising from the growth of a single cell clone (for example a hybridoma, a eukaryotic host cell transfected with a DNA molecule coding for the homogeneous antibody, a prokaryotic host cell transfected with a DNA molecule coding for the homogeneous antibody, etc.) and is generally characterized by heavy chains of one and only one class and subclass, and light chains of only one type. Monoclonal antibodies are highly specific and are directed against a single antigen. An "antigen" is a predetermined molecule to which an antibody can selectively bind. The target antigen may be a polypeptide, a carbohydrate, a nucleic acid, a lipid, a hapten or any other naturally occurring or synthetic compound. Preferably, the target antigen is a polypeptide.

A typical antibody is comprised of two light chains and two heavy chains that are joined by disulfide bonds. As used in the invention, the term "light chain" refers to mammalian immunoglobulin light chain, lambda (A) or kappa (κ), having two successive domains: one constant domain and one variable domain. As used in the invention, the term "heavy chain" refers to chain of mammalian immunoglobulin denoted by: alpha (α), delta (δ), epsilon (ε), gamma (γ), and mu (µ). Each heavy chain has two regions, the constant region and the variable region. The constant region is identical in all antibodies of the same isotype. The variable region of each heavy chain is composed of a single Ig domain. The "variable region" or "variable domain" of an antibody refers to the amino-terminal domains of the heavy or light chain of the antibody. The variable domain of the heavy chain may be referred to as "V_{H}". The variable domain of the light chain may be referred to as "V_{L}". These domains are generally the most variable parts of an antibody and contain the antigen-binding sites. Each variable region contains three segments called "complementarity-determining regions" ("CDRs") or "hypervariable regions" ("HVRs"), which are primarily responsible for binding an epitope of an antigen and are interspersed with regions that are more conserved, designated "Framework Regions" (FR). The CDRs thus direct the specificity of the binding of the antibody. They are usually referred to as CDR1, CDR2, and CDR3, numbered sequentially from the *N*-terminus.

Each VH and VL is composed of three CDRs and four FRs, arranged from amino-terminus to carboxy-terminus in the following order: FR1, CDR1, FR2, CDR2, FR3, CDR3, FR4. The assignment of amino acid sequences to each domain is in accordance with well-known conventions (for example, the IMGT unique numbering convention as disclosed by Lefranc, M.-P.,et al., Dev. Comp. Immunol., 27, 55-77 (2003)). The functional ability of the antibody to bind a particular antigen depends on the variable regions of each light/heavy chain pair, and is largely determined by the CDRs. The variable region of the heavy chain differs in antibodies produced by different B cells, but is the same for all antibodies produced by a single B cell or B cell clone (or hybridoma). By contrast, the constant regions of the antibodies mediate the binding of the immunoglobulin to host tissues or factors, including various cells of the immune system (e.g. effector cells) and the first component (Clq) of the classical complement system.

The term "chimeric antibody" as used herein refers to an antibody containing a natural variable region (light chain and heavy chain) derived from an antibody of a given species in combination with constant regions of the light chain and of the heavy chain of an antibody of a species heterologous to said given species. Thus, a "chimeric antibody", as used herein, is an antibody in which the constant region, or a portion thereof, is altered, replaced, or exchanged, so that the variable region is linked to a constant region of a different species, or belonging to another antibody class or subclass. "Chimeric antibody" also refers to an antibody in which the variable region, or a portion thereof, is altered, replaced, or exchanged, so that the constant region is linked to a variable region of a different species, or belonging to another antibody class or subclass. Such chimeric antibodies, or fragments of same, can be prepared by recombinant engineering. For example, the chimeric antibody could be produced by cloning recombinant DNA containing a promoter and a sequence coding for the variable region of a non-human monoclonal antibody of the invention, notably murine, and a sequence coding for the human antibody constant region. A chimeric antibody according to the invention coded by one such recombinant gene could be, for example, a mouse-human chimera, the specificity of this antibody being determined by the variable region derived from the murine DNA and its isotype determined by the constant region derived from human DNA.

As used herein, the term "humanized antibody" refers to a chimeric antibody which contain minimal sequence derived from non-human immunoglobulin. In one embodiment, a humanized antibody is a human immunoglobulin (recipient antibody) in which residues from a CDR of the recipient are replaced by residues from a CDR of a non-human species (donor antibody) such as mouse, rat, rabbit or non-human primate having the desired specificity, affinity, and/or capacity. In some instances, framework ("FR") residues of the human immunoglobulin are replaced by corresponding non-human residues. Furthermore, humanized antibodies may comprise residues that are not found in the recipient antibody or in the donor antibody. These modifications may be made to further refine antibody performance, such as binding affinity. In general, a humanized antibody will comprise substantially all of at least one, and typically two, variable domains, in which all or substantially all of the hypervariable loops correspond to those of a non-human immunoglobulin sequence, and all or substantially all of the FR regions are those of a human immunoglobulin sequence, although the FR regions may include one or more individual FR residue substitutions that improve antibody performance, such as binding affinity, isomerization, immunogenicity, etc. The number of these amino acid substitutions in the FR are typically no more than 6 in the Heavy (H) chain, and in the Light (L) chain, no more than 3. The humanized antibody optionally will also comprise at least a portion of an immunoglobulin constant region (Fc), typically that of a human immunoglobulin.

An "antibody fragment" comprises a portion of an intact antibody, preferably the antigen binding and/or the variable region of the intact antibody. Examples of antibody fragments include Fab, Fab', F(ab')₂ and Fv fragments; diabodies; linear antibodies (see U.S. Pat. No. 5,641,870); single-chain antibody molecules and multispecific antibodies formed from antibody fragments. Papain digestion of antibodies produced two identical antigen-binding fragments, called "Fab" fragments, and a residual "Fc" fragment, a designation reflecting the ability to crystallize readily. The Fab fragment consists of an entire L chain along with the variable region domain of the H chain (V_{H}), and the first constant domain of one heavy chain (C_{H}1). Each Fab fragment is monovalent with respect to antigen binding, i.e., it has a single antigen-binding site. Pepsin treatment of an antibody yields a single large F(ab')₂ fragment which roughly corresponds to two disulfide linked Fab fragments having different antigen-binding activity and is still capable of cross-linking antigen. Fab' fragments differ from Fab fragments by having a few additional residues at the carboxy terminus of the C_{H}1 domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known. The term "Fv" as used herein refers to the minimum antibody fragment which contains a complete antigen-recognition and -binding site. This fragment consists of a dimer of one heavy- and one light-chain variable region domain in tight, non-covalent association. From the folding of these two domains emanate six hypervariable loops (3 loops each from the H and L chain) that contribute the amino acid residues for antigen binding and confer antigen binding specificity to the antibody. However, even a single variable domain (or half of an Fv comprising only three hyper variable regions specific for an antigen) has the ability to recognize and bind antigen, although at a lower affinity than the entire binding site. "Single-chain Fv" also abbreviated as "sFv" or "scFv" are antibody fragments that comprise the V_{H} and V_{L} antibody domains connected into a single polypeptide chain. Preferably, the sFv polypeptide further comprises a polypeptide linker between the V_{H} and V_{L} domains which enables the sFv to form the desired structure for antigen binding.

In a preferred embodiment, the antibody or fragment of the invention is multispecific, and in particular bispecific. As such, it can be chosen in the group consisting of: bispecific IgGs, IgG-scFv₂, (scFv)₄-IgG, (Fab')₂, (scFv)₂, (dsFv)₂, Fab-scFv fusion proteins, (Fab-scFv)₂, (scFv)₂-Fab, (scFv-C_{H}2-C_{H}3-scFv)₂, bibody, tribody, bispecific diabody, disulfide-stabilized (ds) diabody, 'knob-into whole' diabody, single-chain diabody (scDb), tandem diabody (TandAb), flexibody, DiBi miniantibody, [(scFv) ₂-Fc] ₂, (scDb-C_{H}3)2, (scDb-Fc)₂, Di-diabody, Tandemab., etc. or any combination.

"Functional fragments" of the antibodies of the invention comprise a portion of an intact antibody, generally including the antigen binding or variable region of the intact antibody or the Fc region of an antibody which retains or has modified FcR binding capability. Examples of antibody fragments include linear antibody, single-chain antibody molecules and multispecific antibodies formed from antibody fragments.

As used herein, the term "derivative" refers to a polypeptide having a percentage of identity of at least 75% with the amino acid sequence of the therapeutic antibody or functional fragment thereof as disclosed previously and having the same activity. Preferably, a derivative has a percentage of identity of at least 75%, at least 80%, at least 85%, at least 90% and more preferably of at least 95% with said amino acid sequence, and preferably of at least 99% with said amino acid sequence.

The identity percentage between said two derivative sequences can be identified by a global alignment of the sequences in their entirety (e.g., when the sequences are of about the same size). This alignment can be performed by means of an algorithm that is well known by the skilled person, such as the one disclosed in Needleman and Wunsch (1970). Accordingly, sequence comparisons between two amino acid sequences or two nucleotide sequences can be performed for example by using any software known by the skilled person, such as the "needle" software using the "Gap open" parameter of 10, the "Gap extend" parameter of 0.5 and the "Blosum 62" matrix.

When local alignment of the sequences is to be considered (e.g., in case of derivatives that have a smaller size than the sequences of the invention), then said alignment can be performed by means of a conventional algorithm such as the one disclosed in Smith and Waterman (J. Mol. Evol. 1981; 18(1) 38-46).

The invention also provides vectors encoding homologous amino acid sequences that are "similar" to the ones recited herein. "Similarity" of two targeted amino acid sequences can be determined by calculating a similarity score for the two amino acid sequences. As used herein, the "similarity score" refers to the score generated for the two sequences using the BLOSUM62 amino acid substitution matrix, a gap existence penalty of 11, and a gap extension penalty of 1, when the two sequences are optimally aligned. Two sequences are "optimally aligned" when they are aligned so as to produce the maximum possible score for that pair of sequences, which might require the introduction of gaps in one or both of the sequences to achieve that maximum score. Two amino acid sequences are substantially similar if their similarity score exceeds a certain threshold value. The threshold value can be any integer ranging from at least 1190 to the highest possible score for a particular reference sequence. For example, the threshold similarity score can be 1190, 1200, 1210, 1220, 1230, 1240, 1250, 1260, 1270, 1280, 1290, 1300, 1310, 1320, 1330, 1340, 1350, 1360, 1370, 1380, 1390, 1400, 1410, 1420, 1430, 1440, 1450, 1460, 1470, 1480, 1490, 1500, or higher. Amino acid substitution matrices and their use in quantifying the similarity between two sequences are well-known in the art and described, e.g., in Dayhoff et al. (1978), "A model of evolutionary change in proteins", "Atlas of Protein Sequence and Structure," Vol. 5, Suppl. 3 (ed. M. O. Dayhoff), pp. 345-352. Natl. Biomed. Res. Found., Washington, D.C. and in Henikoff et al. (1992) Proc. Natl. Acad. Sci. USA 89:10915-10919. While optimal alignment and scoring can be accomplished manually, the process is facilitated by the use of a computer-implemented alignment algorithm, e.g., gapped BLAST 2.0, described in Altschul et al., (1997) Nucleic Acids Res. 25:3389-3402, and made available to the public at the National Center for Biotechnology Information website. To generate accurate similarity scores using NCBI BLAST, it is important to turn off any filtering, e.g., low complexity filtering, and to disable the use of composition-based statistics. One should also confirm that the correct substitution matrix and gap penalties are used. Optimal alignments, including multiple alignments, can be prepared using, e.g., PSI-BLAST, available through the NCBI internet site and described by Altschul et al., (1997) Nucleic Acids Res. 25:3389-3402.

The vector of the invention can contain a detectable marker. This marker confers resistance to the selection and allows cells to stably integrate the plasmid into a chromosome and be expanded into a cell line. Usually this detectable marker is an antibiotic resistance gene, such as ZeocinR or nourseothricin omycin resistance gene. The use of auxotrophic strains of *P*. *tricornutum* can also be used for the selection of microalgae that have been transformed (both stably or transiently). Auxotrophic strains for uracil, tryptophan and histidine have been generated as described in literature, but auxotrophy to leucin or methionine can also be considered. The generation of strains with particular genes inactivated can also allow positive selection of transformed strains. For example, inactivation of the *umps* and *apt* genes allows resistance to 5-FOA (5-Fluoroorotic acid) and 2-FA (2-fluoroadenine) respectively.

### Figure legends

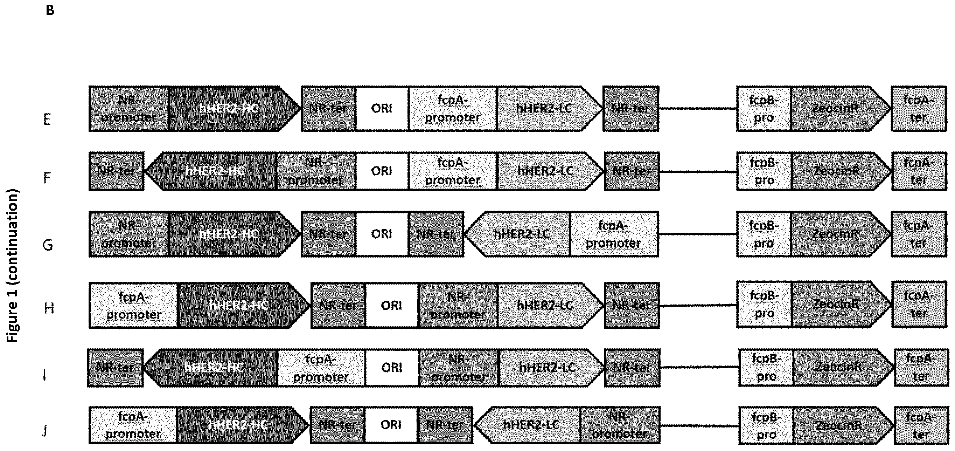

**Figure 1** **- Structure of different vectors according to the invention**
**Figure 2** **- H4-1B and V-ATPase C promoters are able to drive expression of Trastuzumab in the diatom *P. tricornutum.* A.** Design of the constructs used to produce Trastuzumab (TRA) in *P*. *tricornutum* under the control of the H4-1B (Slattery et al., 2018) and the V-ATPase C (Watanabe et al., 2018) promoters. **B.** Expression of trastuzumab under the control of each promoter/terminator couple was assessed by ELISA after 8 and 15 days of culture on at least 29 independent clones. The results are expressed in µg/L . **C.** Output is represented as pg of mAbs produced per cell. Statistical differences between each construction were analysed by a two-way ANOVA using the Tukey's multiple comparisons test. ns corresponds to no statistical difference observed here (p-value > 0.05), one asterisk (*) corresponds to a p-value < 0.05.

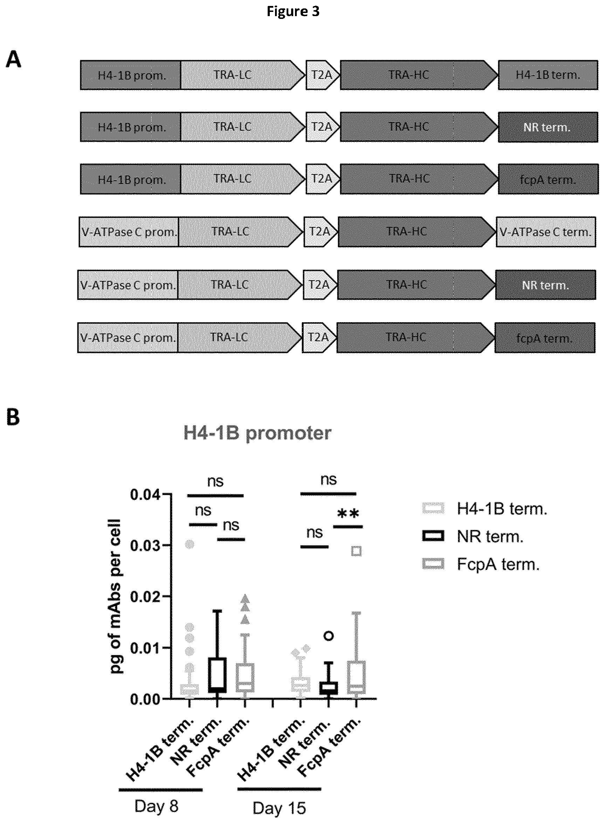

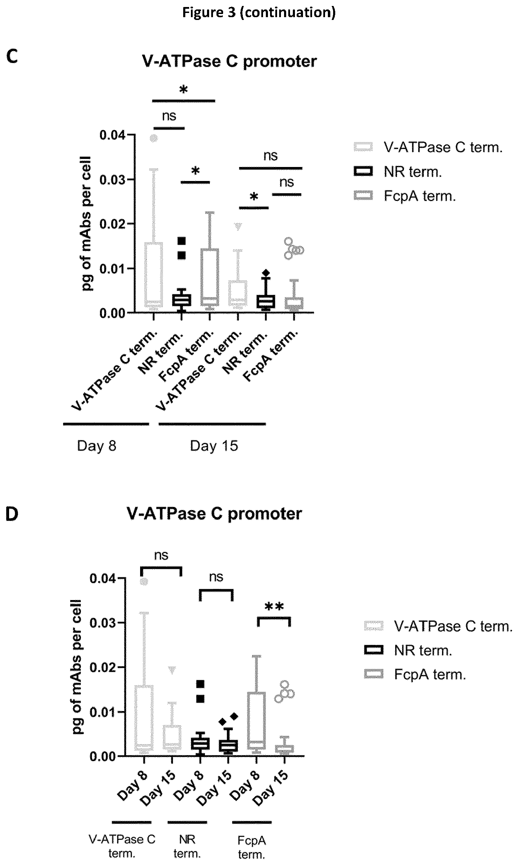

**Figure 3** **- Evaluation of the influence of different terminators on Trastuzumab expression driven by the H4-1B and V-ATPase C promoters in *P*. *tricornutum.* A.** Three different terminators were tested in combination with the H4-1B (Slattery et al. 2018) and V-ATPase C promoters (Watanabe et al., 2018): the native H4-1B terminator, the native V-ATPase C terminator, the NR terminator and the fcpA terminator. B. Expression of trastuzumab under the control of each promoter/terminator couple was assessed by ELISA after 8 and 15 days of culture, respectively, on at least 31 independent clones and is represented as pg of mAbs produced per cell. Statistical differences between each construction were analysed by a two-way ANOVA using the Tukey's multiple comparison test: ns corresponds to a p-value > 0.05; one asterisk (*) corresponds to a p-value < 0.05; two asterisks (**) corresponds to a p-value < 0.005.

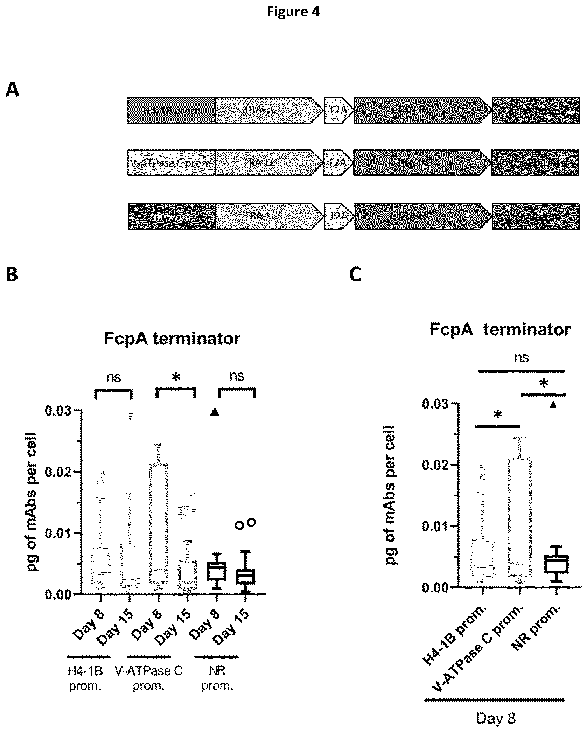

**Figure 4** - **Comparison of HA-1B, V-ATPase C and NR promoter strengths on Trastuzumab expression in *P. tricornutum.*** A. Three different vectors were used to compare the strengths of each promoter in combination with the fcpA terminator. **B.** Expression of Trastuzumab under the control of each promoter was assessed by ELISA after 8 and 15 days of culture, respectively, on 31 independent clones. Output is represented as pg of mAbs produced per cell. Statistical differences between each construction were analysed by a Mann-Whitney-Wilcoxon test: ns corresponds to a p-value > 0.05; one asterisk (*) corresponds to a p-value < 0.05. C. Comparison of the expression of Trastuzumab under the control of each promoter at day 8. ELISA output is represented as pg of mAbs produced per cell. Statistical differences between each construction were analysed by a two-way ANOVA using the Tukey's multiple comparison test: ns corresponds to a p-value > 0.05; one asterisk (*) corresponds to a p-value < 0.05
**Figure 5** - **Confirmation of the better efficiency of the NR promoter/fcpA terminator couple on mAb production yield using the Rituximab. A.** The production yield of Rituximab under the control of NR promoter was evaluated in combination with either the NR or fcpA terminators. **B.** Expression of rituximab under the control of each different promoter was assessed by ELISA after 8 days of culture on 36 independent clones. Output is represented as pg of mAbs produced per cell. Statistical difference was analysed by a Mann-Whitney-Wilcoxon test: one asterisk (*) corresponds to a p-value < 0.05.

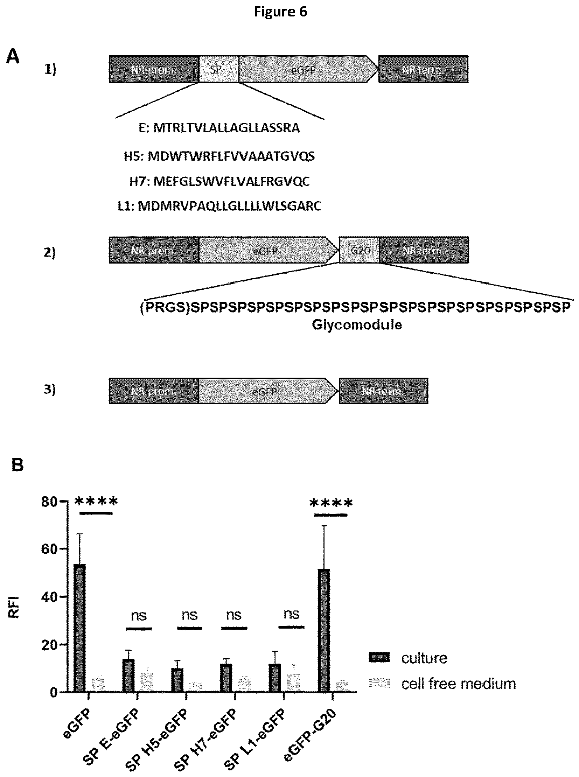

**Figure 6** - **Evaluation of the influence of different heterologous signal peptides (SP) or glycomodule on the secretion of eGFP produced under the control of the NR promoter in P. *tricornutum.* A.** Schematic diagrams of the expression cassettes used. Six different vectors were constructed to evaluate the influence of the heterologous SP E (Kober et al., 2013), H5, H7, L1 (Haryadi et al., 2015) and G20 glycomodule (Ramos-Martinez et al., 2017). The peptide sequences of the different heterologous SP E, H5, H7 and L1 as well as the one from the SP20 *C*-terminal tail are indicated. A SP/G20-less vector was used as a control. **B.** Relative fluorescence intensity of the eGFP measured on an 8-days culture, either on the total culture or on the cell free medium (CFM) after removing the cells. Error bars represent the standard deviation (SD, n= 9). Statistical differences were analysed by a two-way ANOVA using Sidak's multiple comparison's test: ns corresponds to a p-value > 0.05; three asterisks (***) correspond to p-value < 0,0001.
**Figure 7** - **Evaluation of the influence of the different heterologous and endogenous signal peptides (SP) on the production of Trastuzumab and Rituximab under the control of the NR promoter in *P. tricornutum.* A.** Six different vectors were constructed to evaluate the influence of the endogenous SP HASP1 (Erdene-Ochir et al., 2019) and heterologous SP E (Kober et al., 2013), H7, L1 (Haryadi et al., 2015). The SP HASP1 and E were added on both HC and LC of Trastuzumab and Rituximab. The SP L1 was added to LC and H7 to HC also on the two different mAbs. **B.** Secretion of Trastuzumab (T) under the control of each SP was assessed by ELISA after 8 days of culture. Cell free culture media of 36 independent clones were analysed. Output of the ELISA is represented as pg of mAbs produced per cell. Statistical differences were determined using a Mann-Whitney test: ns p-value > 0.05; ** p-value < 0.005; **** p-value <0.0001. **C.** Secretion of Rituximab (R) under the control of each SP was assessed by ELISA after 8 days of culture. Cell free culture media of 36 independent clones were analysed. Output of the ELISA is represented as pg of mAbs produced per cell. Statistical differences between each construction were analysed using a Mann-Whitney test: ns corresponds to a p-value > 0.05; two asterisks (**) corresponds to a p-value < 0.005.

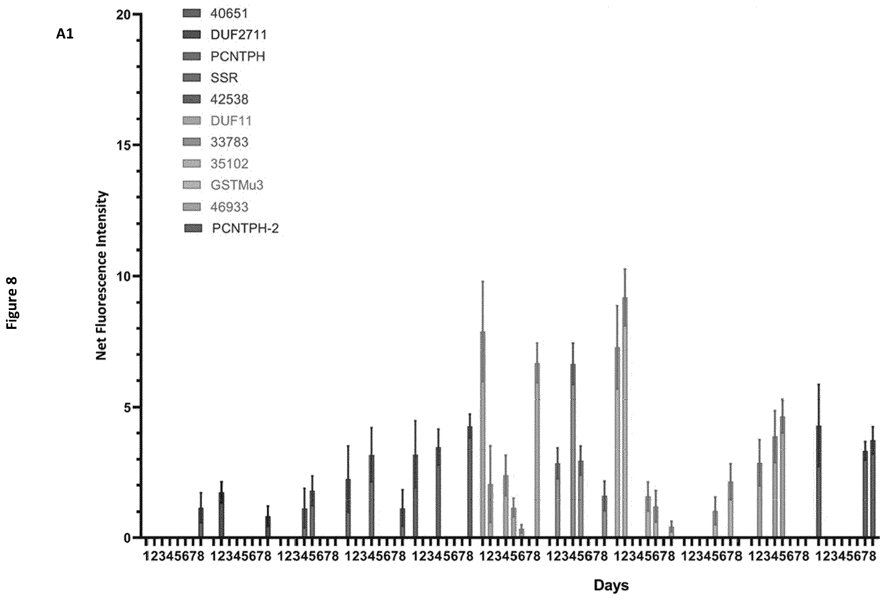

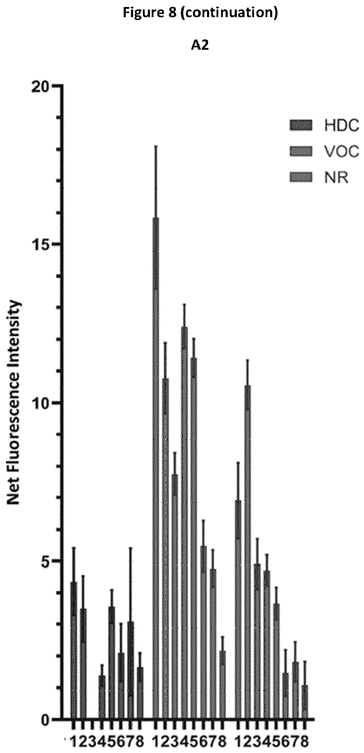

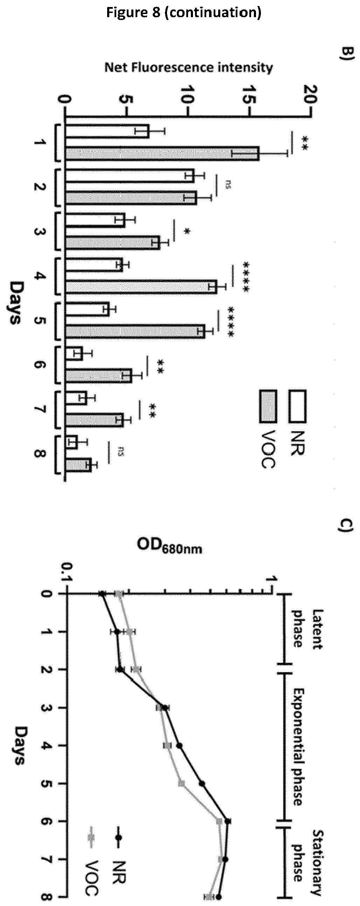

**Figure 8** - **Levels of eGFP expression driven by novel promoters in P. *tricornutum* cells grown under low salinity conditions. A)** Mean of net eGFP fluorescence in transformed *P. tricornutum* cell lines grown over 8 days under low salinity conditions. Only promoters for which eGFP expression could be detected are shown. A1) Promoters allowing a cyclic expression of eGFP; A2) Promoters allowing eGFP expression throughout the culture. **B)** Comparison of eGFP expression driven by the Phatr3_J34085 promoter (VOC) and NR promoter over an 8-day culture in low salinity conditions. **C)** Growth curves of cultures containing the 16 clones of the Phatr3_J34085 (VOC) and NR cell lines over an 8-day period. Error bars represent the standard error of means (SEM, n = 9 for every day except on day 5 where n = 8). Statistical differences were analyzed by a Welsh's t test comparison and by a Mann-Whitney test for data that are following a normal distribution or not (according to Shapiro-Walk test). ns: p-value > 0.05, * p-value < 0,05, **: p-value < 0,01, ***: p-value < 0,001.

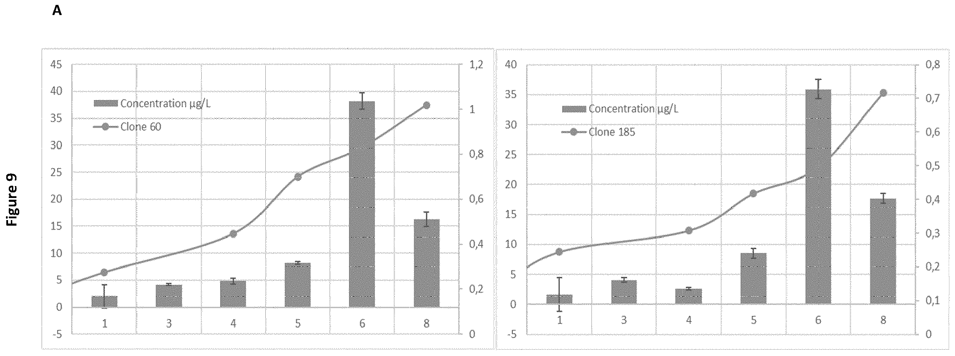

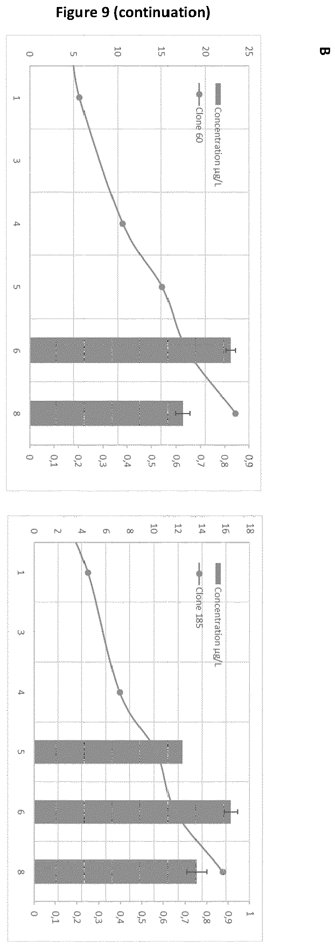

**Figure 9** **- Production of recombinant mAb in two independent clones of *Phaeodactylum tricornutum* cultured in different media: A) the Conway medium containing NO3 as a nitrogen** source and B) the MM medium.

### Examples

### Example 1: INCREASED PRODUCTION OF MONOCLONAL ANTIBODIES IN P. TRICORNUTUM STRAINS

In order to increase the production yield and secretion of these mAbs in *P. tricornutum's* culture medium, different promoter/terminator couples were tested. We demonstrated that the V-ATPase C promoter/FcpA terminator pair could increase the yield of mAbs production in P. *tricornutum* in an 8-day bioprocess. We also highlighted that the use of the FcpA terminator was preferential to the endogenous terminator associated with the promoter for an increase in production yield. In parallel, we tested the influence of different heterologous and endogenous signal peptides on the secretion of antibodies in the culture medium. Surprisingly, the heterologous signal peptide "E" was shown to be more effective than the endogenous one of the HASP1 protein that is the most abundant secreted protein in *P*. *tricornutum's* culture medium. These results contribute to the discovery and characterisation of various key elements to be considered in the vector design in order to increase the production yield of recombinant proteins in *P. tricornutum.*

### 1. Materials and methods

### 1.1. Culture conditions

The strain of *P*. *tricornutum* Pt1.8.6 (CAP 1055/1) were acquired from the Culture Collection of Algae and Protozoa (CCAP, UK). *P*. *tricornutum* was cultivated at 19°C on a photoperiod of 16/ 8h light/ dark cycle with light intensity of 30 µmol photons.m⁻².s⁻¹ either on 1.5-2% agar plates or in liquid cultures under agitation at 150 rpm. Cells were grown in 100 % artificial seawater (33.3 g.L⁻¹ of sea salt (Instant Ocean^{®}, Aquarium System, Sarrebourg, France)) filtered through 0.45 µm filters and sterilized by autoclave. The sterilized culture medium was supplemented with 1 mL.L⁻¹ of Conway NaNO₃ nutritive medium (Na₂EDTA.2H₂O : 45g.L⁻¹; NaNO₃ : 100 g.L⁻¹; H₃BO₃ : 33,6 g.L⁻¹; NaH₂PO₄ : 20 g.L⁻¹; FeCl₃ : 0,768 g.L⁻¹; ZnCl₂: 21 mg.L⁻¹; CoCl₂, 6H₂O : 20 mg.L⁻¹; (NH₄)₆Mo₇O₂₄, 4H₂0: 9 mg.L⁻¹; CuSO₄, 5H₂O : 20 mg.L⁻¹; MnCl₂,4H₂O : 360 mg.L⁻¹; B₁ vitamin : 200 mg.L⁻¹; B₁₂ vitamin : 10 mg.L⁻¹) and 80 mg.L⁻¹ of sodium metasilicate (Na₂SiO₃). The medium supplemented in Conway and sodium metasilicate was named SAS (Supplemented Artificial Seawater) medium. Transformants were grown in a SAS medium where NaNO₃ was replaced by 80.25 g.L⁻¹ of NH₄Cl (SAS-NH₄ medium) as sole nitrogen source. For the transformation steps, 50% artificial seawater was used, but Conway NH₄Cl and sodium metasilicate were used at the same concentration. This medium was named 50% SAS-NH₄ medium. For ELISA experiments, the positive transformants were grown in the Mann and Myers' medium described recently (Song, Lye and Parker, 2020). Indeed, the Mann and Myers' medium contained NO₃. Therefore, in this culture condition, the NR promoter is expressing continuously the recombinant mAbs.

### 1.2. Plasmid construction

Various human signal peptides ("H5", "H7", "L1" from (Haryadi *et al.,* 2015) and "E" from (Kober et al., 2013)) shown in the literature to be effective for the secretion of recombinant mAbs in CHO cells were selected. Their capacity to lead the secretion of recombinant proteins in *P. tricornutum* was first assessed using eGFP as reporter. The addition of a C-terminal 20-unit tandem repeat Serine-Proline glycomodule was also assessed as described for *Chlamydomonas reinhardtii* (Ramos-Martinez, et *al.,* 2017). The eGFP DNA sequence was recovered from the pPhaT1-eGFP vector (Zaslavskaia et *al.,* 2000) and was cloned in the pPha-NR shuttle vector (JN180663.1) optimized by e-Zyvec (Loos, France). This "pPhaNR-eGFP" vector was used as a reference to assess the intracellular fluorescence of eGFP.

DNA sequences from the different signal peptides, glycomodule as well as sequences encoding the Heavy Chain (HC) and Light Chain (LC) of Trastuzumab (Li *et al.,* 2011) and of Rituximab (https://go.drugbank.com/drugs/DB00073) were synthesized and codon optimized for expression in *P. tricornutum.* Signal Peptides and glycomodule nucleotide sequences were cloned respectively either at the N- or C-terminal extremity of eGFP in the pPhaNR-eGFP vector by e-Zyvec. Codon optimized sequences encoding for HC and LC of Trastuzumab and Rituximab were cloned in fusion with the T2A peptide (Szymczak *et al.,* 2004). Different constructs containing either the Nitrate Reductase (NR), the V-ATPase C and H4-1B promoters (Slattery *et al.,* 2018; Watanabe *et al.,* 2018) or different terminators (NR, FcpA, V-ATPase, H4-1B) were tested for their ability to increase expression level of recombinant mAbs like Trastuzumab in *P. tricornutum.* The most promising human signal peptides resulting from the screening step with eGFP, namely L1, H7 and E were evaluated for their capacity to secrete recombinant mAbs in *P. tricornutum.* The secretion of both Trastuzumab and Rituximab under these heterologous signal peptides was analysed. In parallel, the use of the endogenous signal peptide corresponding to the HASP1 protein, the most abundant secreted protein in *P. tricornutum* that was previously used to successfully drive the secretion of eGFP (Erdene-Ochir *et al.,* 2019) was also evaluated for its capacity to secrete Rituximab in *P*. *tricornutum's* culture medium.

### 1.3. P. tricornutum transformation and screening of positive clones

The different plasmids were introduced into diatom cells in exponential growth phase (10⁸ cell per ml) by using the Biolistic Particle Delivery System (PDS)-1000/He (Bio-Rad Laboratories, CA) and a protocol adapted from (Apt et al., 1996). Approximately 2×10⁹ cells of *P*. *tricornutum* were taken from culture in exponential growth phase and centrifuged at 3.000 × g, during 5 minutes at 19°C. Cell pellets were gently resuspended with 150 µl of fresh 50 % SAS-NH₄ medium and were spreading on 2% agar plates containing 50% SAS-NH₄ medium. Cells were stably transformed 24h later by biolistic transformation using 1350 psi rupture disks and M-17 tungsten particles (Biorad, Marnes Ia Coquette, France) coated with 5 µg of plasmid DNA with Biolistic PDS-1000/He. After transformation, cells were incubated in the dark at 19°C. After 48h, cells were plated on 1.5% agar plates containing 50 % SAS-NH₄ medium and 75 µg.ml⁻¹ of Zeocin (Invitrogen). Plates were then incubated at 19 C° during at least two weeks, on a photoperiod of 16: 8h light: dark cycle with light intensity of 30 µmol photons.m⁻².s⁻¹. Isolated Colonies were streaked on agar SAS-NH₄ medium containing 75 µg.mL⁻¹ of Zeocin. This selection step was repeated twice. To confirm the insertion of the transgenes within *P*. *tricornutum* cells, 100 clones of each transformation events were tested by PCR using specific primers **(Table 1).** Cells were resuspended in 30 µL of the following extraction buffer (10mM Tris HCI, 1mM EDTA, 1M NaCl and 1% Nonidet P40 (NP40), pH8) and were heated at 95C° for 10 min. After centrifugation at 3.000 × g for 5 min, supernatants were diluted 5-fold with DNAse-free water and 1 µL was used to perform the PCR. Transgenes were amplified with the DreamTaq DNA polymerase (Promega) and specific primers designed on both sides of the HC and LC chains of the antibodies were used. Specific primers named Tra-F/Tra-R and RTX-F/RTX-R respectively were used to screen transformants that have potentially received constructs containing trastuzumab or rituximab as gene of interest, respectively **(Table 1).** PCR products were analysed by electrophoresis using 1% agarose gel electrophoresis.

**Table 1: Table summarizing the specific primers used for the PCR screening of the P. tricornutum clones.**

| **Primers** | **5'-3' Nucleotides Sequences** | **Hybridization Temperature** |
|---|---|---|
| TRA-F | CGATAATGCGTTGCAGTCGG (SEQ ID NO :68) | 60°C |
| TRA-R | TCGGACCCTTAGTACTCGCA (SEQ ID NO :69) | 60°C |
| RTX-F | ATCTCACGCGTTGAAGCAGA (SEQ ID NO :70) | 61°C |
| RTX-R | CCCTGGCTTCACTAGTTCGG (SEQ ID NO :71) | 61°C |

### 1.4. eGFP screening of transformants

Colonies growing on selective media after biolistic transformation were grown in 96-well plates in 150 µl of SAS-NH₄ medium under agitation (150 rpm) at 19°C on a photoperiod of 16 / 8h light: dark cycle with light intensity of 30 µmol photons m⁻².s⁻¹. Six to seven days after the first inoculation, 50 µl of cultures were recovered and subcultured in 100 µl of fresh SAS-NH₄ medium under the same conditions. In order to allow the activation of the NR promoter and thus the expression of eGFP, NaNO₃ was added to the cells at a final concentration of 1 mM on day six. Two days after induction of the NR promoter, cultures were transferred into a black, clear-bottom 96-well plates (Costar^{®}, Sigma Aldrich, Saint Quentin Fallavier, France). Screening of transformants was performed by exciting eGFP at 485 nm/20nm and emission was measured at 538/20 nm using a fluorometer (FlexStation 3 MultiMode Microplate Reader - Molecular Devices, Tecan Group Ltd, Männedorf, Switzerland). A cut-off filter of 530 nm was applied.

### 1.5. Evaluation of the secretory efficiency of signal peptides

The culture volumes of the top three expressing eGFP clones for each construct were increased to a volume of 50 ml in SAS-NH₄ medium with an initial cell density adjusted to 5×10⁶ cells.ml⁻¹. Cells were grown and induced as described above and harvested on day 8. 150 µl of total culture were recovered and the rest of the culture was centrifuged at 3.000 × g for 5 minutes. Overall culture and supernatant were loaded in a black, clear-bottom 96-well plates (Costar^{®}) and eGFP fluorescence was measured as described above. All measurements were made with three technical replicates on three independent biological replicates. The normality of the data was tested using GraphPad Prism 8, version 8.0.2 prior to an ANOVA analysis using Sidak's multiple comparison test.

### 1.6. Western Blot analyses

For Western Blot analyses, cell cultures of 100 mL were performed and harvested as described above. After centrifugation, the cell pellets were resuspended in 500 µL of lysis buffer (0.1M Tris HCL, pH 7.5 containing 1 tablet of SIGMA*FAST*^{™} Protease Inhibitor Cocktail (SIGMA)). Total proteins were extracted using lysing beads (E-matrix lysing tubes, MP Biomedicals^{®}, Fisher Scientific, Illkirch, France) and grounded for 3 cycles of 30 seconds at 6.5 m.s⁻¹ in a FastPrep-24^{™} homogenizer (MP Biomedicals^{®}). Lysing tubes were centrifuged at 5,000 × g for 5 minutes and supernatant was collected, then 500 µL of lysis buffer was added to the lysing tubes and a second homogenisation step was performed. Secreted eGFP was recovered by precipitating total proteins from the supernatant using a Trichloroacetic acid (TCA)/ NP40 mixture. Briefly, 75 µL of NP40 was added to the supernatant, samples were vortexed and incubated at room temperature for 30 minutes. Then, 2.5 mL of TCA was added and samples were homogenised by vortex and incubated on ice during 1h30. Samples were centrifugated at 10,000 g for 30 minutes at 4°C. Pellets were washed with 500 µL of cold acetic acid three times with a centrifugation step (10,000 × g for 30 min) between each wash. Pellets were left at room temperature (RT) until complete evaporation of acetone.

The presence of eGFP in supernatant or in cell pellets was analysed by Western Blot analysis under reducing conditions (Leammli Buffer containing β-mercaptoethanol heated at 100°C during 10 min). Succinctly, the same amount of each sample was loaded on Tris-Glycine gel (12%), and the migration of proteins was carried out in a Tris-Glycine migration Buffer at 150 V. Purified eGFP produced in plants was used as positive control and Bovine Serum Albumin was used as negative control. One µg of each control was loaded on the SDS-PAGE gel. After migration, proteins were transferred to a nitrocellulose membrane using a wet tank transfer system (Bio-Rad) either during 2 hours at 60 V or overnight at 10 V. After transfer, the membrane was saturated in Tris buffer salt (TBS) complemented with Tween 20 at 0.1% (TBS-T) buffer and 5 % skim milk for at least 2 hours at RT under agitation at 150 rpm. Detection of eGFP was performed by incubating the membrane with a rabbit anti-eGFP polyclonal antibody (Invitrogen, ThermoFisher Scientific, Illkirch, France) diluted at 1: 2,000 in TBS-T buffer with 1 % skim milk for 3 hours at RT. After washing, the membrane was then incubated with Horseradish peroxidase (HRP)-conjugated goat anti-rabbit antibody (Invitrogen) diluted at 1: 30,000 in TBS-T buffer for 1 h30 at RT. Finally, revelation of the membrane was performed using the ECL Pico Plus kit (ThermoFisher Scientific) according to the manufacturer's instructions.

### 1.7. Quantification of the level of expression of Rituximab and Trastuzumab in P. tricornutum culture supernatant

*P. tricornutum* selected positive clones were grown in Mann and Myers' medium (Song, Lye and Parker, 2020). For each set of transformants containing a different construct, at least 30 independent clones were grown in 5 mL of fresh medium under an agitation of 200 rpm at 19C° during either 8 or 15 days. At the end of the culture, an aliquot of 1 mL was collected and centrifuged at 14,000 × g for 5 minutes in order to remove the diatom cells and cell debris. The amount of mAbs present in the *P*. *tricornutum* culture medium was finally quantified by ELISA using the MabTech Human IgG ELISA ^{BASIC} kit (ALP) using the instructions from the manufacturer (MabTech, Sweden). The quantities of mAbs produced in the different conditions were then determined as compared to the standard curve using GraphPad Prism 8. Statistical analyses were performed with GraphPad Prism 8, version 8.0.2. The normality of the data was first tested prior to either an ANOVA analysis using the Tukey's multiparametric comparisons test or a Mann-Whitney test. Details of the tests that have been used are described in the legend of each figure.

### 2. Results

### 2.1. Search for a promotor/terminator couple that allows increasing the production yield of recombinant mAbs in the diatom P. tricornutum

Among the various possibilities of promoter/terminator couples to be tested, we decided to evaluate the capacity of the promoter and terminator of the H4-1B and V-ATPase genes to drive the production of Trastuzumab. These promoters correspond to the promoter of the histone H4 (Slattery *et al.,* 2018) and the one of the vacuolar ATP synthase 16-kDa proteolipid subunit clumping factor A (V-ATPase C), respectively (Watanabe *et al.,* 2018). So far, they have never been used to produce recombinant mAbs. The H4-1B promoter has been described previously to improve by 30% the mRNA relative expression of a reporter gene as compared to other promoters such as 40SRPS8, γ-Tubulin, FcpC and FcpF (Slattery *et al.,* 2018). The activity of the V-ATPase C gene promoter has been demonstrated to increase by approximately 2.73 times the expression of *Sh ble* mRNA transcripts under both light and dark conditions at the stationary phase as compared to the *fcpA* gene promoter (Watanabe *et al.,* 2018). The constructs allowing expression of the Trastuzumab as a single cistron (promoter - TRA-LC - T2A - TRA-HC - terminator) were built in combination with either the H4-1B promoter or the V-ATPase C promoter using their respective terminator sequences **(****Figure 2A****).** These constructs were used to transform *via* biolistic the diatom cells. After PCR screening of the clones on genomic DNA, expression of the mAbs was chosen as an output for the assay. Therefore, we investigated the strength of the promoters by following the amount of protein produced under their control. In agreement, after PCR screening using specific primers, at least twenty-nine independent positive clones containing the gene of interest were analysed by ELISA to evaluate the amounts of recombinant mAbs accumulated in the cell-free culture medium after 8 and 15 days of culture, respectively. These time points were chosen to maximize productivity of the recombinant mAbs as it was reported previously that proteins are mainly expessed at the stationnary phase of cell culture (Erdene-Ochir et al., 2016). Moreover, the efficiency of some promoter such as the V-ATPase C one was assessed in the late stationnary phase (Watanabe *et al.,* 2018).

Data reported in **Figure 2B** demonstrated that both H4-1B and V-ATPase C promoters allow the production of Trastuzumab in *P. tricornutum.* The quantity of mAb produced under the control of the H4-1B promoter reached an average of 84 µg.L⁻¹ at day 8 and 78 µg.L⁻¹ at day 15 **(****Figure 2B****).** In contrast, the quantity of Trastuzumab produced under the control of the V-ATPase C promoter achieved an average of 212 µg.L⁻¹ at day 8 and 112 µg.L⁻¹ at day 15. The difference observed between the two promoters is significant at day 8 as demonstrated by the use of a non-parametric Mann-Whitney test (p-value = 0.0171) **(****Figure 2B****).** In order to perform a fair comparison and avoid differences due to the variation in biomass between the clones, the amount of mAbs produced was normalised for all the experiments relatively to the number of cells per mL. This gave a result expressed in pg of mAbs produced per cell, representing the productivity per diatom cell unit **(****Figure 2C****).** Such a treatment of the data provided stronger results and confirmed at day 8 the significant difference of mAbs produced under the control of the H4-1B promoter (mean of 2.2×10⁻³ pg per cells) and under the control of the V-ATPase C (mean of 9.9×10⁻³ pg per cells), the latest representing a better promoter to drive the expression of mAb at day 8 (4.5 times increase as compared to the H4-1B).

In order to go further and evaluate the influence of the terminators on the activities of the H4-1B and V-ATPase C promoters, we built 6 vectors containing the LC and HC of the Trastuzumab driven either by the H4-1B promoter in combination with the H4-1B, NR or FcpA terminators or by the V-ATPase C promoter tested in combination with the same terminator sequences **(****Figure 3A****).** As shown in **Figure 3B****,** the use of different terminators in combination with the H4-1B promoter did not significantly improve the production yield of Trastuzumab in *P*. *tricornutum* at day 8. Even not significant, the terminator FcpA seemed to rise the quantity of mAb at day 8. In contrast, the amount of mAb produced per cell unit increased significantly at day 15 using the FcpA terminator as compared to the NR terminator (p value = 0,0039, two-way ANOVA Turkey's multiple comparisons test).

When using the V-ATPase C promoter in combination with its endogenous, the NR or the FcpA terminator sequences **(****Figures 3C and 3D****),** the better efficacy of the FcpA terminator was confirmed as it allowed a significative improvement of the mAb quantities secreted by the diatom as compared to the V-ATPase C and NR terminator sequences (above 2-fold increase). Moreover, it gave a better production yield of mAbs at day 8.

Then, we compared the activities of the H4-1B and V-ATPase C promoters to the NR promoter that was previously used for the production of recombinant anti-viral antibodies in *P*. *tricornutum* (Hempel *et al.,* 2011, 2017; Hempel and Maier, 2012). All these promoters were used in combination with the FcpA terminator **(****Figure 4A****)** to drive the expression of the Trastuzumab. At least thirty-one independent clones were analysed for each construct. Their respective strength was first evaluated at day 8 and day 15. Even not always significant using a non-parametric Mann-Whitney test, the three promoters presented higher activities for the production of mAb at day 8 **(****Figure 4B****).** Therefore, we decided to compare their respective performance at day 8 **(****Figure 4C****).** It resulted from this comparison that, at day 8, the H4-1B is the weakest promoter for the production of mAb (Mean = 0.0054 pg of mAbs per cell) as compared to the V-ATPase C and NR promoters (Mean = 0.0094 pg of mAbs per cell), the best one being, in the conditions of culture used being the V-ATPase C promoter leading to a mean of 0.1348 pg of mAb per cell unit (p value = 0.0325 as compared to the H4-1B promoter and p value = 0.0395 as compared to the NR one using a two-way ANOVA multiparametric Turkey's test). Therefore, usage of the V-ATPase C promoter allowed 14- and 25-fold increases as compared the NR and H4-1B promoters, respectively.

In order to validate the observation made for the production of Trastuzumab at day 8 with the FcpA terminator and demonstrate that this is not specific for this mAb and can be extended to other mAb candidates, we expressed the Rituximab under the control of the NR promoter in combination with either the NR or FcpA terminator sequences **(****Figure 5A****).** The expression of Rituximab was analysed by ELISA in thirty-six independent clones at day 8. The median of the mAbs quantity produced per diatom cell was 0,6.10³ pg for the vector containing the NR terminator and 1,1.10³ pg for the one containing the FcpA terminator, thus confirming the positive effect of using FcpA terminator. This result was demonstrated to be significant with a p-value of 0,0131 using the non-parametric Mann Whitney test **(****Figure 5B****).**

Altogether, these results demonstrated that the use of the V-ATPase C promoter in combination to the FcpA terminator is the best option to produce recombinant mAbs in *P. tricornutum* for an 8-day bioprocess.

### 2.2. Heterologous signal peptides increase the secretion of eGFP in P. tricornutum

We then evaluated the impact of signal peptides on the secretion of mAbs in the culture medium of *P. tricornutum.* Different signal peptides have been previously described in the literature to increase the secretion of mAbs in CHO cell cultures. Thus, we decided to evaluate the efficiency of such signal peptides for the production and secretion of mAbs in *P*. *tricornutum* cells. Therefore, signal peptides named "H5", "H7", "L1" (Haryadi *et al.,* 2015) and "E" (Kober et al., 2013) were chosen. First, we tested the capacity of the selected heterologous signal peptides to improve the secretion of eGFP in the culture medium of *P*. *tricornutum.* Therefore, different constructs containing eGFP under the control of the NR promoter and terminator were designed with or without the different signal peptides. These constructs are represented in **Figure 6A****.** Another construct containing a glycomodule C-terminal tail rich in Serine-Proline repeating unit (G20) at the end of the eGFP was also evaluated. Such a strategy was previously demonstrated to increase the secretion of Venus in the green microalgae C. *reinhardtii* (Ramos-Martinez *et al.,* 2017). The different constructs were used to transformed *P. tricornutum* cells. Positive transformants were then screened based on their fluorescence intensity that is reflecting the level of eGFP expression. Transformants presenting the highest expression levels were selected for further analysis. Then, the overall fluorescence intensity of a 50 mL culture of the three best expressing clones for each construct was investigated. Finally, the cultures were centrifugated and the fluorescence of the cell-free medium reflecting the eGFP secreted in the culture medium was measured and compared to the fluorescence of the total culture **(****Figure 6B** **and Table 2).**

**Table 2: Relative percentage expressed as percent of eGFP fluorescence measured in the cell-free culture medium of P. tricornutum transformants in comparison to the intracellular fluorescence. The given value represents the mean of three technical replicates for three independent clones for each construct. SP: signal peptide; G20 corresponds to the Glycomodule C-terminal tail containing 20 repeating units of Serine and Proline**

| Constructs | Relative percentage of eGFP fluorescence in the cell free culture medium (%) |
|---|---|
| eGFP | 11.3 |
| SP E-eGFP | 56.5 |
| SP H5-eGFP | 43.7 |
| SP H7-eGFP | 48.2 |
| SP L1-eGFP | 60 |
| eGFP-G20 | 8.5 |

When comparing the intensity of the fluorescence in the culture and in the cell-free medium for the clones expressing the different constructs, it appears that between 43% to 60% of eGFP fluorescence was observed in the culture media. In contrast, as expected, only 11% of the fluorescence appears to be secreted in the cell-free medium in the control experiment of eGFP clones without any signal peptide. The clones expressing eGFP-G20 construct showed predominantly fluorescence inside the cells as observed for the cytosolic eGFP used as a control **(****Figure 6B** **and Table 2).** These results were confirmed by Western Blot using a specific anti-eGFP antibody (data not shown). Altogether, these data demonstrate the ability of heterologous signal peptides to drive the secretion of eGFP in *P*. *tricornutum* cells. Therefore, the best signal peptides namely H7, L1 and E retrieved from this screening step were then used either alone or in combination to express both Trastuzumab and Rituximab in *P*. *tricornutum.*

### 2.3. Heterologous signal peptides increase the secretion of mAbs in P. tricornutum

In order to demonstrate the efficacy of the H7, L1 and E heterologous signal peptides to drive the secretion of mAbs in *P*. *tricornutum* cells, constructs allowing the expression of Trastuzumab and Rituximab were built **(****Figure 7A****)** in parallel to a construct that contained the endogenous signal peptide of the HASP1 protein (Uniprot: B7G4A0). This protein has been described to be the most abundant protein naturally secreted in *P*. *tricornutum* culture medium (Erdene-Ochir *et al.,* 2019). The different constructs were used to stably transformed *P*. *tricornutum* cells. The positive clones were selected by PCR and ELISA. Finally, the amounts of mAbs produced in thirty-two independent clones expressing the Trastuzumab were analysed for the different constructs after 8 days of culture. **Figure 6B** reports on the amount of Trastuzumab produced in the culture medium at day 8 normalised per diatom cell (pg per cell). These results demonstrated that the amount of Trastuzumab secreted in *P. tricornutum's* culture media with the endogenous HASP1 signal peptide is the lowest. The heterologous signal peptide E and the L1/H7 combination used allowed a 1.9-fold improvement for E and a 2.7-fold improvement for L1/H7, respectively by comparison to HASP1. These differences were significant according to a non-parametric Mann-Whitney test (p-value < 0,0001 for the SP E and p-value = 0,0012 for the L1/H7 combination). To confirm these results, similar studies were performed for the Rituximab **(****Figures 7A and 7C****).** Thirty-six independent clones were analysed by ELISA. Again, the highest production yield of secreted Rituximab was obtained with the heterologous signal peptide E **(****Figure 7C****)** which results in a 2.6-fold increase.

### Example 2: PRODUCTION OF TRASTUZUMAB IN DIFFERENT P. TRICORNUTUM STRAINS

### 1. Material and methods

### 1.1. Culture conditions

Prior to the transformation, the ten *P. tricornutum* wild type strain were cultivated at 19°C under a photoperiod of 16h light/8h dark with light intensity of 30 µmol photons m⁻².s⁻¹ either on 1.5 % agar plates or in liquid cultures (glass Erlenmeyer) under agitation at 150 rpm. Cells were grown in 100 % artificial seawater (33,3 g/L of sea salt (Instant Ocean^{®})) filtered through 0,45 µm filters and sterilized by autoclave. The sterilized culture medium was supplemented with 1 mL.L⁻¹ of Conway NaNO₃ nutritive medium (Na₂EDTA.2H₂O : 45g /L; NaNO₃ : 100 g/L; H₃BO₃ : 33,6 g/L; NaH₂PO₄ : 20 g/L; FeCl₃ : 0,768 g/L; ZnCl₂: 21 mg/L; CoCl₂, 6H₂O : 20 mg/L; (NH₄)₆Mo₇O₂₄, 4H₂0 : 9 mg/L; CuSO₄, 5H₂O : 20 mg/L; MnCl₂,4H₂O : 360 mg/L; B1 vitamin : 200 mg/L; B12 vitamin : 10 mg/L) and 80 mg/L of sodium metasilicate (Na₂SiO₃). The medium supplemented in Conway and sodium metasilicate was named SAS (Supplemented Artificial Seawater) medium. Transformants were grown in a SAS medium where NaNO₃ was replaced by 80,25 g.L⁻¹ de NH₄Cl (SAS-NH₄ medium).

Expression of eGFP was carried out on 2 ml cultures in 12 well plates containing 10% artificial seawater supplemented with the same proportions of Conway medium and sodium metasilicate (SAS medium: supplemented artificial seawater). Cultures were grown at 19°C under continuous agitation at 150 rpm on a 16h/8h light/dark cycle.

For antibody expression, *P*. *tricornutum* cells containing the heavy and Light chains transgenes were grown in 5-1000 mL culture inoculated at a density of 0.1-0.6 (OD₆₈₀ₙₘ) in SAS-NH₄ medium at 19°C on a photoperiod of 16h light/8h dark (30 µmol photons m⁻².s⁻¹) for four days. The fifth day, cultures were transferred to 23°C under continuous illumination (37 µmol photons m⁻².s⁻¹). On day six, trastuzumab production was induced by the addition of 1 mM NaNO₃. Cells were harvested two days after induction.

### 1.2. Plasmid construction

DNA sequences encoding for the fully-humanized heavy chain (HC) and light chain (LC) of the monoclonal antibody trastuzumab was retrieved from the patent WO 2011/103700 (Li, Tong and Kan, 2011) and the T2A sequence -EGRGSLLTCGDVEENPGP (SEQ ID NO:9)- from (Szymczak et al., 2004). The specific codon bias of *P*. *tricornutum* was used to synthetize the different genes. Polycistronic vectors containing HC-T2A-LC (vector C), or LC-T2A-HC (vector D) were constructed by e-Zyvec (Loos, France). Briefly, codon-optimized DNA sequences of HC and LC of trastuzumab and T2A were cloned in a modified version of pPha-NR shuttle vector (JN180663.1).

### 1.3. P. tricornutum transformation

### Transformation of P. tricornutum by conjugation

First, an E. *coli* strain containing the pTA-Mob (mobilisation helper) vector was transformed with the SLIC eGFP vector containing the NR promoter. Transformation of *P*. *tricornutum* was adapted from (Karas *et al.,* 2015). Exponentially growing cultures of transformed E. coli (37°C in LB medium) and *P. tricornutum* (19°C in SAS medium) were harvested via centrifugation (10 min at 3 000 g). Cell pellets were resuspended in 500 µl of SOC medium and 50% SAS medium for bacteria and diatoms respectively. 200µl of both prokaryotic and eukaryotic cells were mixed and plated on 1 % agar plates containing 50% SAS medium without silica and 5% LB. Agar plates were incubated at 30°C during 1h30 in dark, then at 19°C on a 16h/8h light/dark cycle for 72 h. Then, 1 mL of 50% SAS medium was added to the plates and cells were scratched and resuspended. 200µl of resuspended cells were plated on selective agar plates containing 50% SAS medium and 20µg.ml-1 of phleomycin. Plates were incubated at 19°C on a 16h/8h light/dark cycle and colonies of transformed *P. tricornutum* appeared approximately 10 and 15 days after the transformation.

Biolistic transformation was adapted and modified from (Apt et al., 1996). Approximately 1×10⁹ cells of *P*. *tricornutum* Pt1.8.6, Pt2,Pt3, Pt4, Pt5, Pt6, Pt7, Pt8, Pt9 and Pt10 strains were taken from culture in exponential growth phase and centrifuged at 3,000 × g, during 5 minutes at 19°C. Cell pellets were gently resuspended with 150 µl of fresh 50 % artificial seawater (16,6 g/L Sea Salt Medium) containing 1 mL.L⁻¹ of Conway NH₄+ medium and were spreading on 2% agar plates containing 50% artificial seawater medium supplemented by 1 mL.L⁻¹ of Conway NH₄⁺. Cells were stably transformed 24h later by biolistic transformation using 1350 psi rupture disks and M-17 tungsten particles coated with 5µg of plasmid DNA using the Particle Delivery System-1000/He (Bio-Rad). After transformation, cells were incubated in the dark at 19°C. After 48h, cells were plated on 1.5% agar plates containing 50% artificial seawater medium supplemented with 1 mL.L⁻¹ of Conway NH₄⁺ medium, 40 mg/L of sodium metasilicate and 75 µg. mL⁻¹ of Zeocin (Invitrogen).

### 1.4. PCR screening of potential transformants

15 days after biolistic transformation, colonies growing on selective medium were subcultured on fresh 1.5% agar plates containing SAS medium with 75µg/ml of Zeocin. Prior to PCR screening, a rapid DNA extraction was performed on approximately 200-250 Zeocin resistant clones. For this purpose, a small amount of biomass from each putative transformant was scraped directly from the agar plates and placed in 30 µl of lysis buffer (1% NP-40, 10 mM Tris HCI, pH 8; 1 mM anhydrous EDTA; 1 M NaCl). Cell lysis was achieved by heating the cells at 95°C for 10 min. Then, lysed cells were vortexed briefly and 120 µl of DNase-free water were added. DNA extractions were maintained at 4°C until performing PCR. PCR amplification was performed using GoTaq^{®} G2 DNA Polymerase (Promega). 1µl of each DNA extraction was used and manufacturer's recommendations were adapted to a final volume of 20 µl. The presence of both HC and LC in transformants that potentially received vector 1 was confirmed using primers 5'-CTGTCATTATAAGTCGGGGGG-3' (SEQ ID NO:72) and 5'-TCGCGACTAATCGTGAAACGG-3' (SEQ ID NO:73) for HC and primers 5'-AAACTCCTCTATCCTCACCTG-3' (SEQ ID NO:74) and 5'-CAACTTTAGTCCCTTGACCG-3' (SEQ ID NO:75) for LC. For transformants that potentially received vector 2, the presence of both chains was confirmed using a single pair primers 5'-CAGCAAGGACTCCACATACAGC-3' (SEQ ID NO:76) and 5'- TCGCGACTAATCGTGAAACGG-3' (SEQ ID NO:77). Amplicons were analyzed on 1.5% agarose gels.

### 1.5. Samples preparation and ELISA assay

For the ELISA assay, cell cultures were adjusted to the same density (OD₆₈₀ₙₘ = 0,6) and samples were harvested 48 hours after induction. Cells were centrifuged (3 000 × g, 5 min) and resuspended in 500 µl of lysis buffer (0,1M Tris HCL, pH 7.5 containing 1 tablet of SIGMAFASTTM Protease Inhibitor Cocktail (SIGMA)) and the supernatant was cleared of residual cells by a second centrifugation step (10 000 × g, 5min) and collected. Total proteins were extracted using lysing beads (E-matrix lysing tubes, MP Biomedicals^{®}) and grounded for 3 cycles of 30 seconds at 6,5 m/s in a FastPrep-24TM homogenizer (MP Biomedicals^{®}). Lysing tubes were centrifuged at 5 000 × g for 5 and supernatant was collected, then 500 µ! of lysis buffer was added to lysing tubes and a second homogenization step was performed.

Samples were analyzed by ELISA on the day of harvest using the Human IgG ELISABASIC kit (MabTech). Briefly, Nunc^{®} MaxiSorpTM plates were coated with 100 µl per wells of MT145 antibody diluted at 2 µg/ml in PBS (pH 7.4) and incubated overnight at 4°C. The following day, plates were washed twice with 200 µl per wells of PBS (pH 7.4), then saturated with 200 µl per wells of PBST (PBS (pH 7.4) with 0,05% Tween 20) and 0,1% BSA and then incubated for 1 hour at room temperature (RT). Plates were washed five times with 200µL per wells of PBST, then 100 µl of samples or standard dilution (ranging from 500 to 2.5 ng/ml) were added and plates were incubated for 2 hours at RT. Plates were washed as described above and 100 µl per wells of MT78-ALP antibody diluted at 1:1000 in PBST containing 0.1% BSA were added, and plates were incubated for 1 hour at RT. Plates were washed as previously described and 100 µl per wells of pNPP solution at 5 mM, and plates were incubated 45 min at 37°C in dark and plates were read at 405nm using a plate reader. Medium and wild-type cells pellets served as a negative control and all measurements were carried out on 3 biological replicates and technical triplicates for each sample.

### 1.6. Purification of the trastuzumab biosimilar

For further functional analysis, the trastuzumab biosimilar was purified using protein-A sepharose. The culture medium was collected as described above, filtered through a 0.22 µm filter as described in (Hempel et al., 2012) then diluted with ultrapure water (v/v) and finally purified through protein-A sepharose (GE HealthCare). After washing with 10 column volumes (CV) of Tris 0.1 M (pH 8). The antibody was eluted with 5 CV of 0.1 M of Glycine-HCI buffer, pH 2.5 into a glass test-tube containing 0.25 CV of Tris buffer 1M, pH 9. The entire purification step was performed at 4°C.

### 1.7. Western Blot

Correct antibody assembly and purification validation were performed by Western Blot analysis under native (Leammli Buffer without β-mercaptoethanol) or reducing conditions (Leammli Buffer with β-mercaptoethanol at 100°C during 10 min). Succinctly, the same amount of each sample was loaded on Bis-Tris (4-12%) or Tris-Glycine gel (12%), and the migration of proteins was carried out in a MOPS-Buffer or a Tris-Glycine migration Buffer respectively at 130 V. Commercial trastuzumab from Roche was used as positive control (under natives or reducing conditions) and BSA was used for negative control. 100 ng of each control was loaded on gel. After migration, proteins were transferred to a nitrocellulose membrane using a wet tank transfer systems (Bio-rad) either during 2 hours at 60 V or overnight at 10 V. After transfer membrane was saturated in TBS-T (TBS with 0,1% Tween 20) buffer with 5 % skim milk for at least 2 hours at RT. Detection of the full-length antibody or heavy and light chains was realized by incubating the membrane with a biotin-conjugated goat anti-human IgG (H+L) (Invitrogen) diluted at 1/3 000 in TBS-T buffer with 1 % skim milk for 3 hours at RT. The membrane was then incubated with HRP-conjugated streptavidin (Sigma) diluted at 1/30 000 in TBS-T buffer for 1h30 at RT. Finally, revelation was performed using the ECL Pico Plus kit (ThermoFisher) according to the manufacturer's instructions.

A glycosylation analysis was also performed using eastern blots revealed by specific lectins prior to mass spectrometry analyses. After antibody loading under reducing conditions, followed by migration and transfer as described above, nitrocellulose membranes were saturated in TBS-T buffer and then incubated in TBS-T buffer containing either 5µg/ml of Concanavalin A (Vector Laboratories) containing 10 mM CaCl₂, a rabbit anti-xylose antibody (Agrisera) or a rabbit anti-fucose antibody (Agrisera) for 3 hours at RT. Membranes were subsequently incubated with HRP-conjugated streptavidin (Sigma) or HRP-conjugated goat anti-rabbit antibody (Invitrogen) respectively, diluted at 1/30 000 in TBS-T buffer for 1h30 at RT. Revelation was performed as described above.

### 2. Results

Different expression vectors were designed to drive the expression of TrastuzumAb under the control of the NR promoter with or without the T2A linker between the genes encoding for both heavy (H) and light (L) chains of TrastuzumAb **(****Figure 1A** showing the structure of the tested vectors A, B, C and D). After the clone selection by PCR, the amounts of mAbs produced in the culture media and within the *P*. *tricornutum* cells were determined using an ELISA assay. It is to note that for such mAbs quantification, no purification or desalting step was introduced. Ten independent clones of *P*. *tricornutum* expressing the same construct were studied. Two different PHAEO strains were studied (Pt1.8.6 and Pt3).

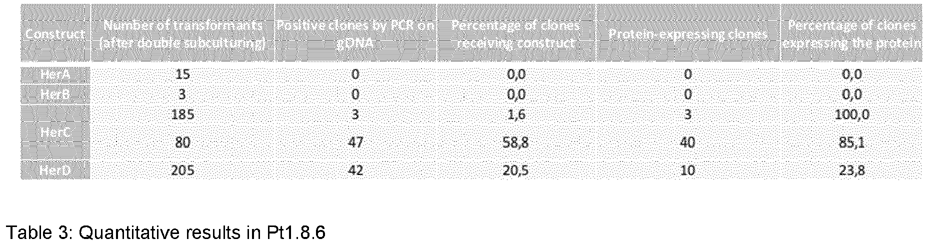

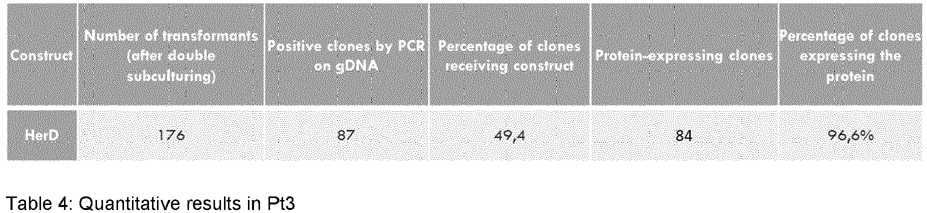

In average for clones expressing the vector D, the mAb production yield is above 20 µg/L for strain Pt1.8.6 using 100 mL of an 8-day culture and 4 g/L in average for Pt3 strain. It is to note that for the latest, the production yield was only measured for the screening stage (5 ml cultures where the culture conditions are not optimal).

### Example 3: IDENTIFICATION OF NEW ENDOGENOUS PROMOTERS OF P. TRICORNUTUM

The selection of a strong promoter that drives high yields of mAbs is a prerequisite for developing a cost-effective expression system. To date, only few endogenous promoters have been characterized in *P*. *tricornutum.* In the present work, we analysed our previously published transcriptomic data from *P. tricornutum* Pt3 strain and identified thirty-three potential "strong" endogenous promoters. First, these putative promoter sequences were cloned and fused to the coding sequence of the enhanced green fluorescent protein (eGFP). Their strengths were assessed by measuring eGFP fluorescence, thus reflecting the level of eGFP expression. Among the thirty-three promoters, thirteen were able to drive eGFP expression. Best results were obtained with the *Phatr3_J34085* (VOC) promoter which allowed a significant increase expression of eGFP by comparison to the one induced by the NR promoter. These findings contribute to the discovery of new genetic tools that could be used in future studies to improve recombinant protein production in *P. tricornutum.*

### 1. Material and methods

### 1.1. Cell culture and growth conditions

The *Phaeodactylum tricornutum* strain Pt3 (CCAP 1052/1B) was used for this study. Cells were grown at 19°C on a 16h/8h light/dark cycle. Screened transformants were maintained on 1,5% agar plates containing 100% artificial seawater (33,3 g.L⁻¹ of sea salt (Instant Ocean^{®})) filtered on 0,45 µm filters and sterilized by autoclave. The sterilized culture medium was supplemented by 1ml.L⁻¹ of Conway medium (Na₂EDTA.2H₂O : 45g.L⁻¹; NaNO₃: 100g.L⁻¹; H₃BO₃: 33,6 g.L⁻¹; NaH₂PO₄: 20g.L⁻¹; FeCl₃ : 0,768 g.L⁻¹; ZnCl₂: 21mg.L⁻¹; CoCl₂, 6H₂O : 20 mg.L⁻¹; (NH₄)₆Mo₇O₂₄, 4H₂0 : 9mg.L⁻¹; CuSO₄, 5H₂O : 20 mg.L⁻¹; MnCl₂,4H₂O : 360 mg.L⁻¹; B1 vitamin : 200 mg.L⁻¹; B12 vitamin : 10 mg.L⁻¹) and 80mg.L⁻¹ of sodium metasilicate.

Expression of eGFP was carried out on 2 ml cultures in 12 well plates containing 10% artificial seawater supplemented with the same proportions of Conway medium and sodium metasilicate (SAS medium: supplemented artificial seawater). Cultures were grown at 19/16°C under continuous agitation at 150 rpm on a 16h/8h light/dark cycle.

### 1.2. Regulatory sequences selection

In order to identify putative promoter sequences, we used the results of a RNA-seq transcriptomic analysis of the three main morphotypes of *P*. *tricornutum* Pt3 strain (Ovide *et al.,* 2018). A selection of the 55 most overexpressed genes (Log2 Fold Change values > 5) in the oval morphotype compared to the other two morphotypes was performed. In silico analyses of these 55 genes and their upstream regulatory sequences were performed using the Integrative Genomic Viewer (IGV v2.11.4) and Blast2GO software in order to retain only the sequences that present higher probability to be promoter regions. Transcriptomic data were visualised using IGV to identify and eliminate mispredicted sequences. Blast2GO software was used to verify the sequence description, and sequences for which no annotation could be made, not even "predicted proteins' were also removed from the study.

### 1.3. Isolation of putative promoter regions

DNA extraction from Pt3 cell line was carried out using the Nucleospin Plant II kit (Macherey-Nagel) following the manufacturer's instructions. Putative promoter sequences were amplified by PCR from Pt3 genomic DNA using the Phusion Green Hot Start II High Fidelity DNA Polymerase (ThermoFisher Scientific). Amplicons were purified using "Wizard SV Gel and PCR Clean-Up System" (Promega) and cloned into either the pJET1.2/blunt cloning vector (ThermoFisher Scientific) or in the Smal linearised pUC19 vector (ThermoFisher Scientific). Cloning into the pJET1.2/blunt cloning vector was realised following the instructions provided by the 'CloneJET PCR Cloning Kit" (ThermoFisher Scientific) and cloning into the pUC19 was realised using T4 DNA ligase (Promega). Cloning vectors were used to transformed DH5α chemically competent *Escherichia coli* (Invitrogen) and colony PCR was performed using the GoTaq G2 polymerase (Promega) and primers as described above. Plasmids were purified from E. coli cells using the Nucleospin Plasmid Kit (Macherey-Nagel). Finally, the putative promoter sequences were verified by Sanger sequencing prior to further experiments (Eurofins, France).

### 1.4. Transformation vectors construction

The pPtpuC3 vector backbone (Karas *et al.,* 2015) required for *P*. *tricornutum* transformation by conjugation was modified by insertion of a "SLIC-eGFP-NRt" transcriptional unit (TU), in order to allow a rapid cloning of the different promoters to be tested in this study. Based on prior work, the TU was amplified from the vector "pPhaNR-eGFP" (Mekhalfi et al., 2022) using the primers 5'-GCT-CTA-GAT-AGT-TGG-AAT-GGT-ACG-TAC-CAA-CTC-CAT-AAG-GAT-CCA-TGG-TGA-GCA-AGG-GCG-AGG-AGC-3' (SEQ ID NO:78) and 5'- ACA-TGC-ATG-CTC-CGG-ATG-CGT-TCA-TTT-TAG-ATC-CTG-ATC-CG-3' (SEQ ID NO:79). After digestion of the pPtpUC3 vector backbone and of the cassette "SLIC-eGFP-NRt" TU with the restriction enzymes Sphl and Xbal, the cassette was ligated into the pPtPuc3 vector using T4 DNA polymerase (Promega) thus allowing the creation of the pPtPUc3_SLIC-eGFP-NRt vector.

For the construction of final vectors, the pPtPUc3_SLIC-eGFP-NRt vector was digested with the restriction enzyme SnaBI (Promega), and the putative promoter regions were amplified from the previously sequencing-validated vectors. The putative upstream regulatory sequences were cloned into the pPtPUc3_SLIC-eGFP-NRt vector using the sequence-and ligation-independent cloning method (SLIC) by adapting the previously described method (Jeong *et al.,* 2012). The insert: vector ratio was increased to 10:1 and 2 units of T4 DNA polymerase (Promega) were used. All the resulting SLIC vectors (empty or promoter containing) constructed were also checked by sequencing as described above for validation prior to *P*. *tricornutum* transformation (Eurofins, France).

### 1.5. Transformation of P. tricornutum

First, an E. *coli* strain containing the pTA-Mob (mobilisation helper) vector were transformed with the SLIC vectors (empty or promoter containing). Transformation of *P*. *tricornutum* was adapted from (Karas *et al.,* 2015). Exponentially growing cultures of transformed E. coli (37°C in LB medium) and *P. tricornutum* (19°C in SAS medium) were harvested via centrifugation (10 min at 3 000 g). Cell pellets were resuspended in 500 µl of SOC medium and 50% SAS medium for bacteria and diatoms respectively. 200µl of both prokaryotic and eukaryotic cells were mixed and plated on 1 % agar plates containing 50% SAS medium without silica and 5% LB. Agar plates were incubated at 30°C during 1h30 in dark, then at 19°C on a 16h/8h light/dark cycle for 72 h. Then, 1 mL of 50% SAS medium was added to the plates and cells were scratched and resuspended. 200µl of resuspended cells were plated on selective agar plates containing 50% SAS medium and 20µg.ml-1 of phleomycin. Plates were incubated at 19°C on a 16h/8h light/dark cycle and colonies of transformed *P. tricornutum* appeared approximately 10 and 15 days after the transformation.

### 1.6. Confirmation of the presence of transgene by PCR

After transformation, colonies growing on selective medium were subcultured on fresh 1,5% agar plates containing SAS medium with 75µg/ml of Zeocin. Prior to PCR screening, a rapid DNA extraction was performed on approximately 20 Zeocin resistant clones. For this purpose, a small amount of biomass from each individual clone was scraped directly from the agar plates and placed in 30 µl of lysis buffer (1% NP-40, 10 mM Tris HCI, pH 8; 1 mM anhydrous EDTA; 1M NaCl). Lysis was achieved by heating cells at 95°C for 10 min. Then, lysed cells were vortexed briefly and 120 µl of DNase-free water were added. DNA extractions were maintained at 4°C until further use. PCR amplification was performed using GoTaq^{®} G2 DNA Polymerase (Promega). 1µl of each DNA extraction was used and manufacturer's recommendations were adapted to a final volume of 20 µl. The presence of the gene encoding eGFP in transformants was confirmed using primers 5'-ATCATGGCCGACAAGCAGAA-3' (SEQ ID NO:80) and 5'-GACTGGGTGCTCAGGTAGTG-3' (SEQ ID NO:81). Amplicons were analysed on 1,5% agarose gels.

### 1.7. Assessment of the activity of potential promoter sequences by eGFP fluorescence intensity measure.

For the same construct, between 7 to 16 independent clones were grown together in pools in 6-well plates containing 5mL of culture. 150µl of each culture was sampled every day to measure cell density (OD680nm) and eGFP fluorescence for 8 days. Measurements were carried out in black, clear-bottom 96-well plates (Costar^{®}). Screening of transformants was performed by exciting eGFP at 485 nm/20nm and emission was measured at 538/20 nm using a fluorometer (FlexStation 3 MultiMode Microplate Reader- Molecular Devices). A cut-off filter of 530 nm was applied. The eGFP fluorescence emitted under the control of each promoter was normalized to a one OD unit (considering the OD680nm linearity region). The normalized fluorescence of the empty vector control (pPtPUc3_SLIC-eGFP-NRt) was subtracted from each condition in order to ignore the chlorophyll background signal. All measurements were made with three technical replicates on three independent biological replicates.

### 1.8. Statistical analyses

Mean values of the net eGFP fluorescence per OD680nm unit, mean values of the growth curve, standard error of the means (SEM) and statistical analyses were calculated and performed using Prism software. The normalised net eGFP fluorescence intensity of the VOC promoter was compared to the values of the reference NR promoter, over the 8 days period of culture. The Shapiro-Walk test was used to assess whether the data were normally distributed or not. The difference between the values obtained at different days for the NR and VOC promoters were evaluated using a Welsh's t-test comparison for values following a normal distribution and by a Mann-Whitney test for values not following a normal distribution.

### 1.9. In silico analyses of validated promoter sequences

Sequences that drove eGFP expression were further analysed in silico. The prediction of cisregulatory elements in these sequences was performed using the PlantCARE database. Additional analysis was performed on promoter that allowed a higher eGFP expression than that obtained with the NR promoter to characterise the binding sites of TFs whose genes have already been identified in P. tricornutum39 using PlantPAN2.0.

### 2. Results

### 2.1. Identification and cloning of potential promoter sequences

*In silico* analyses of the most overexpressed fifty-five genes under salt stress conditions (10% sea water) in the oval morphotype of *P*. *tricornutum* were performed using the Integrative Genomic Viewer (IGV) and Blast2GO software. This allows identifying the potential promoter sequences possessing a good probability to drive transgene expression. A total of 22 sequences were identified as mispredicted because of the lack of consistency between transcriptomic data and the gene prediction. These mispredicted sequences were retrieved for further analysis. The Blast2GO software was used to attribute a functional annotation to the 55 selected genes. Eight out of the 55 genes (14.5%) still do not have any functional annotation. Altogether, these *in silico* analyses reduced the number of potential promoters to analyse to thirty-three (Table 5).

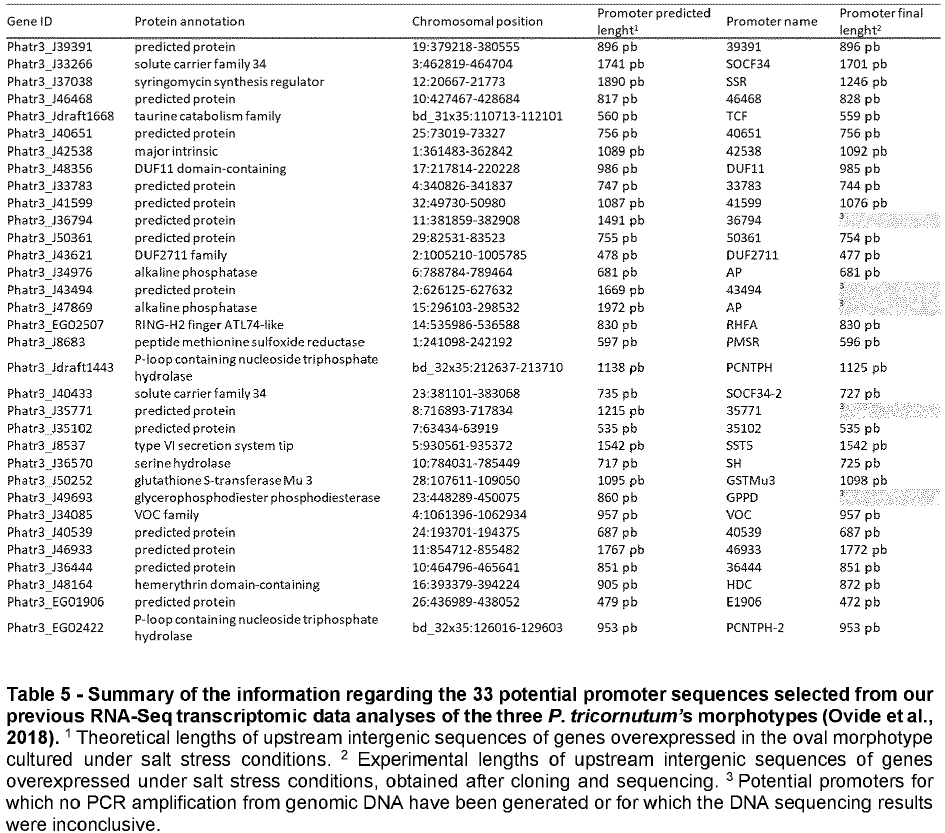

In this study, we considered the entire intergenic region between the STOP codon of the upstream gene and the ATG of the gene of interest as being the putative promoter. The sequences of the thirtythree putative promoters were retrieved from the ASM15095v2 version of the *P. tricornutum* CCAP 1055/1 genome available on the EnsemblProtists website (https://protists.ensembl.org/index.html). PCR amplification of these putative promoters was only possible for twenty-eight of them. Finally, the 28 potential promoter sequences that could be amplified and validated by sequencing exhibited lengths ranging from 477 bp to 1701 bp **(Table 5).**

### 2.2. Construction of transformation vectors

In order to create a versatile vector that would allow a fair comparison of the 28 different potential promoters, we prepared a plasmid vector compatible with the method called (Sequence and) Ligation Independent Cloning ((S)LIC). For this purpose, the pPtpuC3 transformation vector used for episome expression in *P. tricornutum* (Karas *et al.,* 2015) was modified to introduce a 'SLIC-eGFP-NRt' transcriptional unit (TU). This TU contains the eGFP coding sequence, the NR terminator and a "SLIC" cassette allowing the promoters to be cloned easily. As this cloning technique is based on the 3' exonuclease activity of T4 DNA polymerase, complementary ends must be created in the transformation vector and the fragment to be inserted. The twenty-eight cloned potential promoters validated by sequencing were thus amplified from the cloning vector by polymerase chain reaction (PCR) with specific primers. The inducible NR promoter previously used for the production of mAbs in *P. tricornutum* was also amplified from the pPha-NR vector and cloned in the SLIC vector. In this study, the NR promoter was used as a reference in order to select promoters presenting a strength higher than the one of the NR. To overcome the biolistic transformation "position effect" and the variability in copy number of the transgene inserted into the genome, we used episomal expression as recently reported for the characterisation of new promoters (Slattery *et al.,* 2018; Windhagauer *et al.,* 2021). Indeed, compared to biolistic transformation, episomal expression limits the variability of gene expression between clonal lines that received the same construct. After the transformation, *P*. *tricornutum* colonies started to appear on selective medium between 10 to 15 days after the transformation. After two subcultures on fresh agar medium with selective antibiotic, about twenty transformants were screened by PCR with primers specific to the eGFP reporter gene. For almost all transformed cell lines, 16 positive clones in PCR were used to assess the ability of the promoter to express eGFP. For cell lines that received the 42538 and DUF11 promoters, only 12 and 7 clones were used respectively due to a lower number of colonies grown on selective medium. Since the promoters tested in this study were isolated from genes overexpressed under salt stress conditions, *P. tricornutum* cells were cultivated in hyposaline medium (10% sea water) from colonies isolated on agar plates. In order to induce the NR promoter under comparable conditions, the cell line that received this reference promoter were placed in hyposaline medium containing NaNO₃ to activate this nitrate inducible promoter from the beginning of the culture.

### 2.3. Levels of eGFP fluorescence

The fluorescence of eGFP emitted in the different transgenic lines was measured by fluorometry. For a fair assessment of the promoter strength, up to 16 clones that received the same transgene were pooled and grown together for fluorometry analysis. Indeed, even if conjugation is supposed to limit the variability of gene expression between clones, difference in the fluorescence level of a reporter protein reaching up to 30-fold has been reported between two independent clones from the same conjugation event (Windhagauer *et al.,* 2021). Promoter-less vector (pPtPUc3_SLIC-eGFP-NRt) was used to ensure that the observed fluorescence results specifically from the expression of eGFP driven by one of the tested promoter and not from residual background due to chlorophyll. To this purpose, fluorescence emitted by the pool of cell line which was transformed with promoter less vector was subtracted from the fluorescence emitted by each cell line pool. The mean net fluorescence of each transformed cell line pool is presented as normalized fluorescence net per OD₆₈₀ₙₘ unit **(Figure 8A).** Among the 28 potential promoters tested, these results showed that promoters SSR (Phatr3_J37038, SEQ ID NO:29), 40651 (Phatr3_J40651), 42538 (Phatr3_J42538, SEQ ID NO:36), DUF11 (Phatr3_J48356, SEQ ID NO:37), 33783 (Phatr3_J33783), DUF2711 (Phatr3_J43621), PCNTPH (Phatr3_Jdraft1443, SEQ ID NO:30), 35102 (Phatr3_J35102, SEQ ID NO:31), GSTMu3 (Phatr3_J50252, SEQ ID NO:32), VOC (Phatr3_J34085, SEQ ID NO:33), 46933 (Phatr3_J46933), HDC (Phatr3_J48164, SEQ ID NO:34) and PCNTPH-2 (Phatr3_EG02422, SEQ ID NO:35) **(Table 5)** are able to drive eGFP expression in addition to the NR promoter.

In order to compare the efficiency of each promoter during *P*. *tricornutum* growth curve, measurement of the fluorescence intensity of each cell line pool was performed over a period of 8 days. As shown in **Figures 8A** **and** **8B****,** the expression of eGFP driven by the NR promoter, our experimental reference, increased until it reaches its maximum on day-2 after culturing the diatom cells in the presence of NaNO₃. This step is followed by a decrease in eGFP fluorescence from day-3 onwards. The expression of eGFP then continued to reduce until it stabilised at its lower level in the stationary phase.

In contrast, some of the promoter candidates only allowed to drive a low-level expression of eGFP compared to the NR promoter and moreover this expression is observed during a single day. This is the case for promoters: 40651, DUF2711 and PCNTH for which fluorescence intensity is low and only detectable at day-8, day-2, and day-5 respectively. The SSR, 42538, DUF11, 33 783, 35102, 46933, HDC and PCNTH-2 promoters allowed a more cyclic and variable expression of eGFP but mostly at an intensity lowerthan the eGFP fluorescence under the control of the NR promoter. Finally, the VOC promoter (Phatr3_J34085, SEQ ID NO:33) seemed to stand out from the others and exhibited a better promoter activity than the NR one on the basis of eGFP expression **(Figure 8A).**

### 2.4. Comparison of eGFP expression under the control of the VOC and NR promoters

To further compare the activity of the VOC promoter *versus* the NR promoter over time, statistical analyses were performed using a Wesh's t-test comparison for data following a normal distribution and a Mann-Whitney test for samples with data not following a normal distribution. The NR promoter seemed to have a preferential activity in the latent phase with a maximum intensity at day-2. From day-3 onwards, eGFP expression decreased until it stabilised in the stationary phase from day-6 **(****Figures 8B and 8C****).** In contrast, eGFP expression under the control of the VOC promoter did not correlate with the different phases of the growth curve **(****Figures 8B and 8C****).** Indeed, as soon as cells are cultured in a salt-stress condition, the VOC promoter is active. After a maximum expression on day-1, the fluorescence intensity decreased until day-3 before reaching again a maximum value on day-4. From day-6 onwards, eGFP expression slightly decreased again until reaching the same intensity as of the one induced by the NR promoter at day-8. These data are in agreement with previous transcriptomic results reported in the DiatOmicBase (https://www.diatomicsbase.bio.ens.psl.eul) showing that the VOC gene (*Phatr3_J34085* gene; **Table 5)** is largely under-expressed (Log2FoldChange : -4,55) in the stationary phase compared to the early growth phase. Interestingly, according to the DiatOmicBase, nitrate and phosphate starvation or exposition to dark does not seem to influence the expression of the gene VOC.

### 2.5. In silico analyses of promoters driving eGFP expression

Transcription factors (TFs) have been shown to be key elements for the regulation of gene expression. Therefore, an *in silico* analysis was performed to identify the potential *cis*-acting regulatory elements present on the 13 promoters that allowed eGFP expression. The PlantCARE tool was used to identify numerous CAAT-box or TATA-box motifs. These motifs belonging to the minimal promoter to drive expression were found in all promoter sequences allowing to drive eGFP expression. Numerous motifs involved in light response were also identified in all promoters. In contrast, low temperature response motifs were only identified for the SSR, 33783, GSTMu3, HDC and PCNTPH-2 promoters. Surprisingly, putative stress response motifs were not found in all promoters that drove eGFP expression.

Finally, we have deeply investigated the VOC promoter sequence and the binding sites of TFs. The analysis was performed using the "Promoter Analysis" tools available on PlantPAN2.0. Numerous binding sites for four of the five most reported TFs in *P*. *tricornutum* were identified (data not shown). Thus, the VOC promoter exhibited binding sites for the Myb, bZIP (basic leucine zipper), Zinc- finger (C2H2 type) and bHLH (basic Helix-loop-helix) TFs which represent respectively an average of 17.9%, 11.8%, 5.7% and 3.7% of the TFs identified in *P. tricornutum* (Rayko *et al.,* 2010; Thiriet-Rupert *et al.,* 2016). Surprisingly, no binding sites for heat shock factors (HSFs) were identified, although they represent major TF family (30-35%) in *P*. *tricornutum* (Rayko *et al.,* 2010; Thiriet-Rupert *et al.,* 2016) Several cis-acting elements involved in a stress response have been identified in the sequence of the VOC promoter (data not shown).

### Example 4: OPTIMIZATION OF THE GROWTH PARAMETERS OF P. TRICORNUTUM

### 1. Material and methods

Cell cultures were inoculated at approximately 3×10⁶ cells/ml and grown at 19°C under constant agitation (150 rpm) and under a photoperiod of 16h light/8h dark during 8 days. The cultures were performed either in sea water supplemented with Conway or Conway supplemented or F/2. For comparison, the MM medium was also used for the production of the mAbs **(Table 6).**

**Table 6: detailed composition of the culture medium used to produce mAbs in P. tricornutum.**

| Compounds | Concentration in final solution (SAS) | | | | | | I | |
|---|---|---|---|---|---|---|---|---|
| | Conway | | Conway supplemented | | f/2-SI | | M&M | |
| | Mass concentratio n (g.L⁻¹) | Molar concentration (M) | Mass concentration (g.L⁻¹) | Molar concentration (M) | Mass concentration (g.L⁻¹) | Molar concentration (M) | Mass concentration (g.L⁻¹) | Molar concentration (M) |
| Instant Ocean Salt | 33 | | | | | | | |
| Sodium chloride | | | | | | | 5 | |
| Magnesium sulfate heptahydrate | | | | | | | 1,2 | |
| Potassium Chloride | | | | | | | 0,6 | |
| Calcium chloride | | | | | | | 0,3 | |
| Ethylenediaminetetra acetic acid disodium salt dihydrate | 0,045 | 1,21E-04 | 0,045 | 1,21E-04 | 0,00436 | 1,20E-05 | 0,03 | 8,92E-05 |
| Sodium nitrate or Ammonium chloride | 0,1 0,08025 | 1,18E-03 | 0,47 | 5,53E-3 8,79E-3 | 0,075 | 8,83E-04 | 1 | 1,18E-02 |
| Boric acid | 0,0336 | 5,43E-04 | 0,0336 | 5,43E-04 | | | 0,006 | 9,70E-05 |
| Sodium phosphate monobasic | 0,02 | 1,67E-04 | 0,3 | 2,50E-03 | 0,005 | 0,036-e3 | 0,1 | 5,74E-04 |
| *Tris hydrochloride* | | | | | | | 1 | 6,35E-03 |
| Iron(III) chloride | 0,000768 | 4,74E-06 | 0,000768 | 2,84E-06 | 0,00315g/L | 1,00E-05 | 0,002 | 7,19E-06 |
| Zinc chloride | 0,021 | 1,54E-07 | 0,021 | 1,54E-07 | | | | |
| Cobalt(II) chloride hexahydrate | 0,020 | 8,41E-08 | 0,020 | 8,41E-08 | 0,01mg/L | 4,50E-08 | 0,000077 | 2,40E-08 |
| Ammonium molybdate tetra hydrate | 0,009 | 7,28E-09 | 0,009 mg/L | 7,28E-09 | | | | |
| Sodium molybdate dihydrate | | | | | 0,0063 | 3,06E-08 | | |
| Copper(II) sulfate pentahydrate | 0,020 | 8,01E-08 | 0,66 | 2,64E-06 | 0,098 | 3,92E-08 | 0,00002 | 8,01E-09 |
| Zinc sulfate heptahydrate | | | | | 0,022 | 7,65E-08 | 0,33 | 1,15E-06 |
| Manganese(II) chloride tetrahydrate | 0,36 | 1,82E-06 | 0,36 | 1,82E-06 | 0,18 | 9,10E-07 | 0,0014 | 1,11E-05 |
| Thiamine Hydrochloride (Vitamin B1) | 0,2 | 5,93E-07 | 0,2 | 5,93E-06 | 0,1 | 2,96E-07 | | |
| Cyanocobalamin (Vitamin B12) | 0,01 | 7,38E-09 | 0,01 | 7,38E-09 | 0,0005 | 3,69E-07 | | |
| Biotin (Vitamin B7, H) | | | | | 0,0005 | 2,05E-06 | | |

### 2. Results

Both the Conway medium containing NO3 as a nitrogen source and the MM medium allowed the production of the recombinant mAb in PHAEO in two independent clones (Figure 9

## Claims

1. A single polynucleotide vector expressing a therapeutic monoclonal antibody or a functional fragment or derivative thereof, said vector sequence comprising:
a) nucleotide sequences encoding, in the same or in two different Open Reading Frame(s):
- the light chain of said therapeutic antibody, fragment or derivative thereof, preceded at its *N*-terminal end by a first heterologous secretion signal peptide,
- the heavy chain of said therapeutic antibody, fragment or derivative thereof, preceded at its N-terminal end by a second heterologous secretion signal peptide,
said light and heavy chain being separated by a nucleotide sequence encoding a self-cleaving linker,
b) at least one promoter operatively linked with the sequences encoding said light and heavy chains, said promoter being able to drive the expression of said light and heavy chains in the microalgae *Phaeodactylum tricornutum,*
c) at least one translation termination sequence, and
d) optionally, at least one detectable marker.

2. The polynucleotide vector of claim 1, wherein said heterologous secretion signal peptide is chosen among: E of SEQ ID NO:1, H5 of SEQ IDNO:2, H7 of SEQ ID NO:3, B of SEQ ID NO:85 or L1 of SEQ ID NO:4.

3. The polynucleotide vector of claim 1 or 2, wherein said first and second heterologous secretion signal peptides are different, preferably L1 and H7 respectively.

4. The polynucleotide vector of claim 1 or 2, wherein said first and second heterologous secretion signal peptides are identical, and preferably the signal peptide B of SEQ ID NO:85 or the signal peptide E of SEQ ID NO:1.

5. The polynucleotide vector of any of claims 1 to 4, wherein said first and second heterologous secretion signal peptides are followed by a sequence encoding a cleavage site.

6. The polynucleotide vector of any of claims 1-5, wherein said linker is chosen in the group consisting of: E2A (SEQ ID NO:6), F2A (SEQ ID NO:7 or SEQ ID NO:12), T2A (SEQ ID NO:9), P2A (SEQ ID NO:8), IRES (SEQ ID NO:16 or 17), preferably in the group consisting of: T2A and F2A.

7. The polynucleotide vector of any of claims 1-6, wherein said promoter is chosen in the group consisting of: the promoter of the *Nitrate Reductase* gene from *Phaeodactylum tricornutum* (SEQ ID NO:26), the promoter of the *FcpA* gene from *Phaeodactylum tricornutum* (SEQ ID NO: :27), the promoter of the *FcpB* gene from *Phaeodactylum tricornutum* (SEQ ID NO:28), the promoter of the *V-ATPase-C* gene (SEQ ID NO:25), the promoter of the *histone H4-1B* gene (SEQ ID NO:24), the promoter of the *Phatr3_J37038* gene (SEQ ID NO:29), the promoter of the *Phatr3_Jdraft1443* gene (SEQ ID NO:30), the promoter of the *Phatr3_J35102* gene (SEQ ID NO:31), the promoter of the *Phatr3_J50252* gene (SEQ ID NO:32), the promoter of the *Phatr3_J34085* gene (SEQ ID NO:33), the promoter of the *Phatr3_J48164* gene (SEQ ID NO:34), the promoter of the *Phatr3_EG02422* gene (SEQ ID NO:35), the promoter of the *Phatr3_J42538* gene (SEQ ID NO:36), and the promoter of the *Phatr3_J48356* gene (SEQ ID NO:37).

8. The polynucleotide vector of any of claims 1-7, wherein said translation termination sequence is chosen among : the termination sequence of the *Nitrate Reductase* gene (SEQ ID NO:20) or the termination sequence of the *FcpA* gene of *Phaeodactylum tricornutum* (SEQ ID NO:19), the termination sequence of the *V-ATPase-C* gene (SEQ ID NO:22), the termination sequence of the *γ-tubulin* gene (SEQ ID NO:X), the termination sequence of the *Phatr3_J34085 gene* (SEQ ID NO:63) and the termination sequence of the *histone H4* gene (SEQ ID NO:21).

9. The polynucleotide vector of any of claims 1-8, wherein said promoter is the promoter of the *Nitrate Reductase* gene of SEQ ID NO:26 or the promoter of the *V-ATPase-C* gene of SEQ ID NO:25 or the promoter of the *Phatr3_J34085* gene of SEQ ID NO:33 and said translation termination sequence is the termination sequence of the *FcpA* gene of *Phaeodactylum tricornutum* of SEQ ID NO:19.

10. The polynucleotide vector of any of claims 1-9, containing, in this order:
- the promoter of *V-ATPase-C* of SEQ ID NO:25 or the promoter of the *Phatr3_J34085* gene of SEQ ID NO:33,
- the sequence encoding the secretion peptide signal E of SEQ ID NO:1,
- the sequence encoding the light chain of said therapeutic antibody, fragment or derivative thereof,
- the sequence encoding the linker T2A of SEQ ID NO:15,
- the sequence encoding the secretion peptide signal E of SEQ ID NO:1,
- the sequence encoding the heavy chain of said therapeutic antibody, fragment or derivative thereof,
- the termination sequence of the *FcpA* gene of *Phaeodactylum tricornutum* of SEQ ID NO:19.

11. A transformed cell of *Phaeodactylum tricornutum* comprising the polynucleotide vector of any of claims 1-10, said cell being preferably from the strain Pt1, Pt3, Pt4, Pt5 or Pt7.

12. Use of the transformed cell of claim 11, for the production and the secretion of a therapeutic antibody, a functional fragment or derivative thereof.

13. An *in vitro* method for producing a therapeutic antibody, a functional fragment or derivative thereof, said method comprising:
(i) culturing the transformed cell of claim 11 in appropriate culture conditions,
(ii) harvesting the extracellular medium of said culture, and
(iii) purifying the therapeutic antibody, fragment or derivative thereof, which is secreted in said extracellular medium.

14. The method of claim 13, wherein said appropriate culture conditions involve the use of a culture medium containing a nitrate concentration comprised between 0,1g/L and 1g/L and low salt concentration comprised between 5 to 30%.

15. The polynucleotide of claims 1-10, the cell of claim 11, the use of claim 12 and the method of claims 13-14, wherein said therapeutic antibody is a chimeric, humanized, or human anti-viral, autoimmune or anticancer antibody or any variant of this, preferably chosen from the group consisting of: rituximab, trastuzumab, adalimumab, bevacizumab, infliximab, cetuximab, motavizumab, palivizumab, alemtuzumab, dinutuximab, naxitamab, but also comprising for instance, benralizumab, catumaxomab, daratumumab, elotuzumab, epratuzumab, farletuzumab, galiximab, gemtuzumab ozogamicin, ibritumomab tiuxetan, lumiliximab, necitumumab, nimotuzumab, ocrelizumab, ofatumumab, oregovomab, pertuzumab, tositumomab, zalutumumab, and zanolimumab, more preferably Trastuzumab or Rituximab, dinutuximab and naxitamab or derivatives.
